# FASCICULE DE BREVET EUROPEEN

(11) **EP 1 423 539 B1**
(45) Date de publication et mention de la délivrance du brevet: **24.10.2007**
(21) Numéro de dépôt: 02774913.4
(22) Date de dépôt: 09.09.2002
(51) Int. Cl.: C12Q 1/68

(54) **PROCEDE DE DETECTION SUR BIOPUCE**
NACHWEISVERFAHREN MIT BIOCHIP
DETECTION METHOD WITH BIOCHIP

(30) Priorité: 07.09.2001 FR 0111630
(43) Date de publication de la demande: 02.06.2004
(73) Titulaire: BIOMERIEUX, 69280 Marcy-L'Etoile (FR); COMMISSARIAT A L'ENERGIE ATOMIQUE, 75015 Paris (FR)
(72) Inventeur: NOVELLI-ROUSSEAU, Armelle, F-38180 Seyssins (FR); MOUTIN, Séverine, F-38760 Varces Allières & Risset (FR); GINOT, Frédéric, 38120 Saint Egrève (FR); MASSE, Dominique, F-38500 Coublevie (FR)
(74) Mandataire: Guerre, Dominique
(86) Numéro de dépôt international: PCT/FR2002/003052
(87) Numéro de publication internationale: WO 2003/023061

(56) Documents cités:
- WO-A-97/27328
- US-A- 5 599 668
- US-A- 5 753 444
- US-A1- 2001 014 449
- ANDREW TATON T ET AL: "Scanometric DNA Array Detection with Nanoparticle Probes" SCIENCE, AAAS. LANCASTER, PA, US, vol. 289, 8 août 2000 (2000-08-08), pages 1757-1760, XP002158394 ISSN: 0036-8075 cité dans la demande
- HE, L. ET AL.: "Colloidal Au-enhanced surface plasmon resonance for ultrasensitive detection of DNA hybridization" JOURNAL OF THE AMERICAN CHEMICAL SOCIETY, vol. 122, 2000, pages 9071-9077, XP002206375 cité dans la demande

## Description

La présente invention concerne un procédé de lecture, un procédé de détection et un procédé de quantification des réactions chimiques ou biologiques réalisées sur un support, support qui peut être constitué par une boîte de Pétri, une plaque de microtitration, une biopuce ou autres.

On entend par biopuce, une puce présentant à sa surface une pluralité de zones de reconnaissance, c'est-à-dire au moins cent zones de reconnaissance, équipées de molécules présentant des propriétés de reconnaissance. Dans la suite du texte, et par abus de langage, le terme biopuce est utilisé indépendamment de la destination de la puce à une analyse chimique ou biologique. Le concept de biopuce, plus précisément de puce à ADN, date du début des années 90. De nos jours, ce concept s'est élargi puisque des puces à protéine commencent à se développer. Il repose sur une technologie pluridisciplinaire intégrant la micro-électronique, la chimie des acides nucléiques, l'analyse d'images et l'informatique. Le principe de fonctionnement repose sur un fondement de la biologie moléculaire : le phénomène d'hybridation, c'est-à-dire l'appariement par complémentarité des bases de deux séquences d'ADN et/ou d'ARN.

La méthode des biopuces repose sur l'emploi de sondes (séquences d'ADN représentant une portion d'un gène ou un oligonucléotide), fixées sur un support solide sur lesquelles on fait agir un échantillon d'acides nucléiques marqués directement ou indirectement avec des fluorochromes.

Néanmoins, il est tout à fait possible d'utiliser d'autres supports plus classiques tels que les boîtes de Pétri, les plaques de microtitration qui comportent un certain nombre de puits séparés, ou autres. On entend par support une surface d'analyse qui ne comporte que quelques zones de reconnaissance, généralement au plus cent zones de reconnaissance. Chaque zone de reconnaissance comporte au moins une molécule présentant des propriétés de reconnaissance.

Dans tous les cas, les sondes, appelées également molécules de reconnaissance, sont positionnées de manière spécifique sur le support ou puce et chaque hybridation donne un renseignement sur chaque gène représenté. Ces informations sont cumulatives, et permettent de détecter la présence d'un gène ou de quantifier le niveau d'expression de ce gène dans le tissu étudié. Après hybridation, le support ou puce est lavé(e), lu(e) par exemple par un scanner et l'analyse de la fluorescence est traitée par informatique.

Le support ou la puce qui sert à fixer les sondes est généralement constitué de surface plane ou poreuse composée de matériaux, tels que :
- le verre, matériau peu onéreux, inerte et mécaniquement stable ; la surface peut être recouverte d'une grille de Téflon qui délimite des zones hydrophiles et hydrophobes,
- les polymères,
- le silicium, et
- les métaux, notamment l'or et le platine.

Néanmoins, il est également possible d'utiliser des particules, par exemple magnétiques, telles que décrites dans les demandes de brevet WO-A-97/34909, WO-A-97/45202, WO-A-98/47000 et WO-A-99/35500 de l'une des Demanderesses.

Pour la fixation des sondes (ou molécules de reconnaissance), on distingue trois grands types de fabrication.

Il y a, tout d'abord, une première technique qui consiste en un dépôt de sondes pré-synthétisées. La fixation des sondes se fait par transfert direct, au moyen de micropipettes, de micro-pointes ou par un dispositif de type jet d'encre. Cette technique permet la fixation de sondes de taille allant de quelques bases (5 à 10) jusqu'à des tailles relativement importantes de 60 bases (impression) à quelques centaines de bases (micro-déposition) :
- L'impression est une adaptation du procédé utilisé par les imprimantes à jet d'encre. Elle repose sur la propulsion de très petites sphères de fluide (volume <1 nl) et à un rythme pouvant atteindre 4000 gouttes/secondes. L'impression n'implique aucun contact entre le système libérant le fluide et la surface sur laquelle il est déposé.
- La micro-déposition consiste à fixer des sondes longues de quelques dizaines à plusieurs centaines de bases à la surface d'une lame de verre. Ces sondes sont généralement extraites de bases de données et se présentent sous forme de produits amplifiés et purifiés. Cette technique permet de réaliser des puces dénommées microarrays portant environ dix mille spots, dit zones de reconnaissance, d'ADN sur une surface d'un peu moins de 4 cm². Il ne faut toutefois pas oublier l'emploi de membranes de Nylon, dites « macroarrays », qui portent des produits amplifiés, généralement par PCR, avec un diamètre de 0,5 à 1 mm et dont la densité maximale est de 25 spots/cm². Cette technique très flexible est utilisée par de nombreux laboratoires. Dans la présente invention, cette dernière technique est considérée comme faisant partie des biopuces. On peut toutefois déposer en fond de plaque de microtitration un certain volume d'échantillon dans chaque puits, comme c'est le cas dans les demandes de brevet WO-A-00/71750 sous priorité du 20 mai et du 6 décembre 1999 et FR00/14896 du 17 novembre 2000 de l'une des Demanderesses, ou déposer au fond d'une même boîte de Pétri un certain nombre de gouttes séparées les unes des autres, selon une autre demande de brevet FR00/14691 du 15 novembre 2000 de cette même Demanderesse.

La deuxième technique de fixation des sondes sur le support ou puce est appelée la synthèse *in situ.* Cette technique aboutit à l'élaboration de sondes courtes directement à la surface de la puce. Elle repose sur la synthèse d'oligonucléotides *in situ* inventée par Edwin Southern, et est fondée sur le procédé des synthétiseurs d'oligonucléotides. Elle consiste à déplacer une chambre de réactions, où se déroule la réaction d'élongation d'oligonucléotides, le long de la surface de verre.

Enfin, la troisième technique est appelée la photolithographie, qui est un procédé à l'origine des biopuces développées par Affymetrix. Il s'agit également d'une synthèse *in situ*. La photolithographie est dérivée des techniques des microprocesseurs. La surface de la puce est modifiée par la fixation de groupements chimiques photolabiles pouvant être activés par la lumière. Une fois illuminés, ces groupes sont susceptibles de réagir avec l'extrémité 3' d'un oligonucléotide. En protégeant cette surface par des masques de formes définies, on peut illuminer et donc activer sélectivement des zones de la puce où l'on souhaite fixer l'un ou l'autre des quatre nucléotides. L'utilisation successive de masques différents permet d'alterner des cycles de protection/réaction et donc de réaliser les sondes d'oligonucléotides sur des spots d'environ quelques dizaines de micromètre carré (µm²). Cette résolution permet de créer jusqu'à plusieurs centaines de milliers de spots sur une surface de quelques centimètres carré (cm²). La photolithographie présente des avantages : massivement parallèle, elle permet de créer une puce de N-mères en seulement 4 x N cycles.

Toutes ces techniques sont bien entendues utilisables avec la présente invention.

Des procédés utilisant de tels supports ou biopuces peuvent essentiellement être utilisés pour:
- la recherche de la présence ou de l'absence d'un agent pathogène, par exemple d'une bactérie dans la viande,
- la recherche de la présence ou de l'absence de mutations. Connaissant la structure moléculaire du gène, on fabrique des oligonucléotides qui représentent toute ou partie complémentaire de ce gène. En présence de l'échantillon biologique, il y aura hybridation entre toute ou partie dudit gène, généralement marqué, par exemple en fluorescence, et les oligonucléotides, et l'image obtenue par fluorescence permet de savoir s'il y a une mutation et en quelle position elle est située. Dans cette application, l'utilisation des puces à ADN est l'équivalent d'un séquençage pour un diagnostic de mutation, avec un énorme avantage en terme de rapidité, et
- la mesure du niveau d'expression de gènes dans un tissu. Le réseau de la puce porte de très nombreuses sondes qui correspondent à l'ensemble des gènes de l'espèce à étudier. On hybride un échantillon, par exemple d'ARNm préalablement amplifiés, qui représente les gènes actifs du tissu. L'analyse par fluorescence permet de connaître le niveau d'expression de chaque gène.

L'efficacité de tels supports et biopuces a été testée sur des systèmes biologiques bien connus tel que la levure (cycle cellulaire, métabolisme respiratoire, fermentation...). La comparaison des résultats obtenus par les puces avec ceux préalablement obtenus par d'autres approches a démontré une concordance pour les gènes dont l'expression était déjà bien connue dans ces systèmes biologiques. Ces travaux ont ainsi permis de valider la technologie des biopuces vis-à-vis des supports plus classiques que nous avons évoqués.

Par ailleurs, des sociétés se lancent dans de nouvelles méthodes permettant aussi la réalisation d'analyses génomiques en parallèle. Ainsi, la technique des microbilles permet de fixer des sondes sur des microsphères portant une « étiquette » ou marque individuelle, et plus précisément un code génétique. Après la réaction, la lecture de cette marque permet d'identifier sans ambiguïté une microbille et donc la sonde se trouvant à sa surface. Les microsphères sont mises en contact avec l'échantillon test marqué par un ou des fluorochrome(s). Le mélange est ensuite analysé par cytométrie en flux qui va, d'une part, identifier chaque bille en fonction de son « étiquette », et d'autre part, mesurer la fluorescence trahissant une réaction d'hybridation effective.

Les puces à ADN ont ouvert la voie à une nouvelle instrumentation en biologie moléculaire qui peut même intégrer différentes étapes d'analyses sous forme miniaturisée, voir à ce propos les demandes de brevet WO-A-00/78452 sous priorité du 22 juin 1999 et FR00/10978 déposée 28 août 2000 de l'une des Demanderesses. De plus, et comme déjà indiqué ci-dessus, le très fort intérêt suscité ces derniers temps par la protéomique, la discipline clef de la post-génomique, s'accompagne de l'émergence du concept de puces à protéine. Celles-ci font également partie des supports selon l'invention.

Les molécules de reconnaissance peuvent être, par exemple, des oligonucléotides, des polynucléotides, des protéines telles que des anticorps ou des peptides, des lectines ou tout autre système du type ligand-récepteur. En particulier, les molécules de reconnaissance peuvent comporter des fragments d'ADN ou d'ARN.

Lorsque le support est mis en contact avec un échantillon à analyser, les molécules de reconnaissance sont susceptibles d'interagir, par exemple par hybridation dans le cas où il s'agit d'acides nucléiques ou par formation d'un complexe dans le cas où il s'agit d'anticorps et d'antigène, avec des molécules cibles présentes dans un échantillon biologique liquide.

Ainsi, en équipant une biopuce d'une pluralité de zones de reconnaissance avec différentes molécules de reconnaissance différentes, où chaque molécule de reconnaissance est spécifique d'une molécule cible, il est possible de détecter et éventuellement de quantifier une grande variété de molécules contenues dans l'échantillon. Il est bien entendu évident que chaque zone de reconnaissance ne comporte qu'un seul type de molécules de reconnaissance identiques entre elles.

L'ensemble support-molécule de reconnaissance-molécule cible peut être détecté par une molécule de détection. L'ensemble support-molécule de reconnaissance-molécule cible-molécule de détection constitue un test en format sandwich. Les tests en format sandwich sont largement utilisés en diagnostic, que ce soit en diagnostics moléculaires, par exemple test ELOSA (Enzyme-Linked Oligo-Sorbent Assay), ou en diagnostics immunologiques, par exemple test ELISA (Enzyme-Linked Immuno-Sorbent Assay). De façon générale, ils comprennent une molécule de reconnaissance, telle qu'une sonde d'acide nucléique ou un antigène (cas d'un sandwich antigène) ou un anticorps (cas d'un sandwich anticorps), qui sert à capturer une cible, qui sera respectivement constituée par une sonde d'acide nucléique ou un anticorps ou un antigène. Cette molécule de reconnaissance est fixée sur support solide de manière connu pour l'homme du métier, par exemple :
- soit par adsorption,
- soit par couplage direct,
- soit par l'intermédiaire d'une protéine intermédiaire, comme par exemple l'avidine ou la protéine A.
- soit par l'intermédiaire de polymères.
L'ensemble molécule de reconnaissance et molécule cible est ensuite détecté par une molécule de détection, qui sera respectivement constituée d'une sonde d'acide nucléique ou un anticorps ou un antigène. Cette molécule de détection porte ou pourra être ultérieurement associée à un marqueur, marqueur qui est nécessaire pour permettre la détection et/ou la quantification. La molécule de détection qu'elle soit ou ne soit pas encore associée à un marqueur sera toujours appelée molécule de détection.

Par la suite, le terme « hybridation » sera associé à la fixation d'un acide nucléique sur un autre acide nucléique, alors que le terme « complexation » sera associé à la fixation d'un anticorps sur un antigène. Par contre le terme « fixation » aura une définition plus large qui pourra concerner à la fois :
- la fixation d'un acide nucléique sur un autre acide nucléique,
- la fixation d'un anticorps sur un antigène, ou
- la fixation d'une molécule biologique sur un support.

Les tests actuellement disponibles sont des tests tels que ceux développés par l'une des Demanderesses pour les dosages immunologiques ou les puces à ADN mises au point par la société Affymetrix ("Accessing Genetic Information with High-Density DNA arrays", M. Shee et al., Science, 274, 610-614. "Light-generated oligonucleotide arrays for rapide DNA séquence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de vingt nucléotides.

Dans le domaine ELOSA, c'est-à-dire dans le domaine de la détection des acides nucléiques, voir à ce propos la demande de brevet déposée par l'une des Demanderesses WO-A-91/19812, on définit de la même façon un oligonucléotide de capture (molécule de reconnaissance), une cible acide nucléique (molécule cible) qui est soit de l'ADN soit de l'ARN, et un oligonucléotide de détection (molécule de détection). Les oligonucléotides de capture et de détection sont complémentaires d'une partie de la cible mais au niveau de régions de la cible qui sont respectivement différentes structurellement et physiquement, de sorte que les oligonucléotides de capture et de détection ne peuvent pas s'hybrider l'un à l'autre.

Que ce soit en diagnostics moléculaires ou en dosages immunologiques, les éléments de détection portent un marqueur qui permet la détection et/ou la quantification de la cible. Par marquage, on entend la fixation d'un marqueur capable de générer directement ou indirectement un signal détectable. Différents marqueurs ont été développés avec l'objectif permanent d'améliorer la sensibilité. Ils peuvent être soit radioactifs, soit enzymatiques, soit fluorescents soit, comme décrit plus récemment, sous la forme de nanoparticules. Ces nanoparticules sont différentes des microparticules, en particulier par leur taille qui reste très inférieure au micron. Du fait qu'elles sont moins triviales, elles feront l'objet d'un exposé plus développé ultérieurement.
- Une liste non limitative de ces marqueurs, permettant de réaliser de l'imagerie à marqueur unique, sera décrite dans la suite de la description.

Des systèmes indirects peuvent aussi être utilisés, comme par exemple des ligands capables de réagir avec un anti-ligand. Les couples ligand/anti-ligand sont bien connus de l'homme du métier, ce qui est le cas par exemple des couples suivants :
- biotine/streptavidine,
- haptène/anticorps,
- antigène/anticorps,
- peptide/anticorps,
- sucre/lectine,
- polynucléotide/complémentaire du polynucléotide,
- séquence successive d'histidines, dite « tag », pour un métal, par exemple le nickel.
Dans ce cas, c'est le ligand qui porte l'agent de liaison. L'anti-ligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de l'une des Demanderesses ou à l'article J. Histochem. Cytochem. 45 : 481-491, 1997.

Par ailleurs, les Demanderesses ont déposé conjointement une demande de brevet PCT/FR00/03359 sous priorité française du 2 décembre 1999 (WO 0140778) sur une technique d'amplification du signal. Néanmoins, celle-ci ne fonctionne pas sur la base des enseignements tirés des documents précédents, qui augmentent le nombre de marqueurs au niveau des sites de fixation desdits marqueurs, mais utilise plutôt un revêtement particulier à la surface du support, revêtement qui est à base d'une couche mince d'un matériau choisi parmi le nitrure de silicium, le carbure de silicium, les oxydes de titane, l'oxyde d'aluminium, ZrO₂, ZrO₄Ti, HfO₂, Y₂O₃, le diamant, MgO, les oxynitrures (Si_{X}O_{Y}N_{Z}), les matériaux fluorés, YF₃, MgF₂. Ces deux techniques peuvent être cumulées.

Comme cité précédemment, les marqueurs peuvent être également sous forme de nanoparticules. Les premières expériences utilisant des conjugués avec des nanoparticules remontent à 1980. Elles étaient motivées par la recherche de techniques de marquage ultrasensibles qui évitent l'emploi de la radioactivité ou l'emploi de marquages enzymatiques qui demandent non seulement du temps mais aussi l'utilisation de réactifs toxiques. Elles ont été largement développées et employées depuis et de nombreux types de particules ont été mises au point, tels que des microparticules, des nanoparticules de latex ou des particules d'or colloïdale ou encore des particules fluorescentes. Par la suite le terme « particules » sera utilisé indifféremment pour des microparticules ou des nanoparticules ou autres particules de tailles différentes. De nombreuses particules sont maintenant commercialisées (Bangs, Milteny, Molecular Probes, Polyscience, Immunicon).

Les expériences de marquage par des nanoparticules ont débuté dans le domaine des dosages immunologiques par J. H. W. Leuvering, P. J. H. M. Thal, M. Van der Waart and A. H. W. M. Schuurs, Sol Particle Immunoassay (SPIA). Journal of Immunoassay 1(1):77-91, 1980.

L'utilisation des nanoparticules a permis l'accès à d'autres technologies comme la microscopie à force atomique pour la lecture des dosages immunologiques, technologie avec laquelle, sur un modèle de détection TSH, un système sandwich monté sur support de silicium utilisant des conjugués anticorps de détection anti-TSH lié à des nanoparticules d'or a permis d'obtenir une sensibilité de l'ordre de 1 pM. A ce sujet, il est possible de se reporter aux deux publications suivantes :
- Agnès Perrin, Alain Theretz, and Bernard Mandrand Thyroïd stimulating hormone assays based on the detection of gold conjgates by scanning force microscopy. Analytical biochemistry 256:200-206, 1998, et
- Agnès Perrin, Alain Theretz, Véronique Lanet, S.Vialle, and Bernard Mandrand Immunomagnetic concentration of antigens and detection based on a scanning force microscopic immunoassay. *Journal of immunological methods* 8313, 1999

L'utilisation des nanoparticules pour la détection des acides nucléiques n'est observée que depuis très récemment. Les travaux réalisés dans le domaine de l'immunologie trouvent leur équivalent dans le domaine des acides nucléiques. Il n'y a pas d'innovation fondamentale ni dans les méthodes de marquage ni dans les méthodes de détection. La plaque ELISA n'existe cependant plus, elle est en effet remplacée par un support plan. Les nanoparticules sont alors détectées par microscopie, par amplification pour la résonance de plasmon de surface, dite technique « biosensor » (ou biocapteur en français), ou par mesure en AFM. Globalement les modèles biologiques restent des modèles simples.

Les travaux de Taton, T. A., Mirkin C. A. et Letsinger R. L. concernant : « Scanometric DNA array detection with nanoparticle probes » Science 2000 Sep.8 ; 289(5485):1757-60, présentent l'utilisation de nanoparticules d'or couplées à des oligonucléotides de détection de 15 bases pour détecter une cible de 27 bases capturée sur une sonde de 12 bases. Le signal de détection est amplifié par un marquage à l'argent. Pour des concentrations en cible élevée de l'ordre de 10 nanoMolaire (nM), la détection des particules se fait à l'oeil en observant le passage à une coloration rose. Pour les concentrations les plus faibles, 100 picoMolaire (pM) par exemple, la détection nécessite l'emploi d'un scanner. La sensibilité atteinte est de l'ordre de 50 femtoMolaire (fM). Les auteurs sont capables de dissocier les nanoparticules de la surface par chauffage ce qui tend à montrer la spécificité' de leur marquage. Néanmoins, selon l'expérience des Demanderesses et du fait des tailles respectives des oligonucléotides de détection (15 bases), des oligonucléotides cibles (27 bases) et des oligonucléotides de capture (12 bases), il doit obligatoirement y avoir dissociation entre lesdites nanoparticules et les oligonucléotides de détection. Ces travaux s'appuient en outre sur un ensemble d'expériences qui ont fait l'objet de plusieurs publications (1996 ; 1997 ; 1997 ; 2000) et de plusieurs demandes de brevet et brevets, tels que WO-A-98/04740.

Ces chercheurs font une dissociation des nanoparticules qui ne distingue pas les nanoparticules associées à une molécule de détection sans mésappariement avec la cible et les nanoparticules associées à une molécule de détection avec mésappariement avec ladite cible. Par la suite, par « dissociation » on entend notamment la séparation entre la molécule de reconnaissance et la molécule cible et/ou la séparation de la molécule cible et de la molécule de détection. Si cette dissociation est thermique, la caractéristique physique mise en jeu est constituée par le point de fusion (Tm) qui correspond à la zone de température où les molécules d'ADN ou d'ARN se dénaturent. Plus précisément, cette température correspond à un état où une population d'oligonucléotides identiques, en présence d'une quantité identique d'oligonucléotides complémentaires, est à 50 % sous la forme double brin, c'est-à-dire appariés, et à 50 % sous la forme simple brin.

Si les marquages par des nanoparticules permettent d'obtenir une bonne sensibilité, il y a néanmoins un compromis à trouver entre l'augmentation du signal spécifique et la diminution du signal non spécifique. Le signal non spécifique est bien sûr limitant pour l'amélioration de la sensibilité et de la dynamique de la méthode. Les auteurs règlent ces problèmes par optimisation des conditions expérimentales. A titre indicatif, certains auteurs (Okano et al.; Anal. Biochem. 202 :120-125 ; 1992) ont optimisé la concentration en oligonucléotides de détection par particules, d'une part, et la concentration en particules dans la solution, d'autre part. Ils parviennent également à diminuer le non spécifique par addition de BSA (bovin serum albumin).

En ce qui concerne l'utilisation des nanoparticules pour amplifier un signal obtenu sur un biocapteur, on observe les travaux de Lin He, Michael D. Musick, Scheila R. Nicewaener, Franck G. Salinas, Stephen J. Benkovic, Michael J. Natan, and Christine D. Keating Colloïdal Au-enhanced surface plasmon resonance for ultrasensitive detection of DNA hybridization. J. Am. Chem. Soc. 122:9071-9077, 2000. Des oligonucléotides de détection (12 bases) conjugués à des nanoparticules d'or colloïdal sont utilisés pour amplifier la détection d'une cible d'acide nucléique de petite taille (24 mères) par résonance de plasmon de surface. Ils obtiennent une sensibilité de 10 pM soit une densité de surface de 8.10⁸ molécules / cm² avec une amélioration de l'intensité de signal d'un facteur de 100000. Ils ont vérifié la spécificité de l'hybridation en dissociant les nanoparticules de la surface soit par chauffage soit par digestion par des enzymes de restriction si le site de restriction est présent sur la séquence hybridée. Dans ce cas, le marquage est utilisé pour amplifier le signal, et la dissociation sert à montrer que le marquage résulte d'une hybridation spécifique.

Il s'agit ici d'une vérification de l'hybridation. Soit les nanoparticules sont séparées par chauffage, auquel cas on se retrouve dans le cas de figure décrit par Taton (2000) et il n'y a aucune spécificité dans l'élimination du marqueur. Soit les nanoparticules sont séparées par des enzymes de restriction, dans ce cas les nanoparticules sont éliminées, par exemple par lavage, tout en restant associées avec tout ou partie de la molécule de détection et/ou de l'hybride. Il y a un caractère orienté de cette séparation, mais sans dissociation ou avec une dissociation partielle des doubles brins.

Parmi les procédés de l'état de la technique utilisant les nanoparticules, le document de Kubitschko S., Spinke J., Bruckner T., Pohl S. et Oranth N. « Sensitivity enhancement of optical immunosensors with nanoparticles.» Anal. Biochem. 253(1):112-122 de 1997 évoque, en diagnostic immunologique, la dissociation des conjugués anticorps par rapport aux antigènes. Cette dissociation est réalisée par l'acide formique qui sert à régénérer un micro-composant qui comporte encore les anticorps pour des utilisations ultérieures du micro-composant.

Il s'agit plus d'un procédé de nettoyage de micro-composant, de ce fait il n'y a aucune discrimination de faite lorsqu'est réalisée la dissociation des conjugués anticorps par rapport aux antigènes éventuellement associés aux nanoparticules.

Le brevet US-A-6,093,370 propose un autre procédé qui a pour objectif de récupérer un ADN capturé sur une puce à ADN. Il s'agit d'une dissociation photothermique réalisée avec un laser infrarouge (IR) à 1053 nanomètres (nm) qui irradie avec des puissances comprises entre 10 et 100mW la région de la puce sur laquelle on veut récupérer l'ADN. L'ADN remis en solution est amplifié par PCR. Les auteurs montrent qu'ils sont capables d'extraire au moins une molécule d'ADN par 169 nm² soit 10⁻¹⁷ mol/ 1000µm².

Néanmoins, ils n'utilisent pas une dissociation physico-chimique pour déterminer les hybridations réelles car spécifiques (vrais positifs) des autres hybridations.

Outre les techniques de dissociation précédemment citées, c'est-à-dire par dénaturation thermique (chauffage), par dénaturation photothermique (laser IR), par digestion (enzymes de restriction), par la chimie (acide formique), on retrouve dans l'état de la technique d'autres méthodes de dissociation des acides nucléiques.

Ainsi, il est possible de modifier la force ionique du tampon d'hybridation pour dissocier l'oligonucléotide de détection de la cible. En effet, le point de fusion des oligonucléotides varie en fonction de la force ionique du tampon. Plus la force ionique du tampon diminue, moins l'oligonucléotide est stable. Il est également possible de combiner l'action de la température avec les conditions de tampon.

Il est également possible d'utiliser des PNA (Peptide Nucleic Acid) ou tout autres molécules utilisées dans l'état de l'art pour effectuer la capture d'un acide nucléique. Les hybrides PNA/oligonucléotides ont la même stabilité thermique quelque soit la force ionique, c'est-à-dire que leur point de fusion ne varie pas en fonction de la force ionique du tampon d'hybridation. Si la sonde de capture est un PNA et la sonde de détection est un oligonucléotide, en se plaçant à basse force ionique, la sonde de détection se dissocie de la cible alors que la cible reste hybridée avec la sonde de capture (PNA).

Il est possible de modifier chimiquement les sondes de détection pour effectuer la dissociation de l'ADN sous certaines conditions. Ces méthodes de dissociation ne concernent pas les particules qui interagissent avec la surface via les propriétés de surface de la nanoparticules et du support.

On peut également modifier une base de l'oligonucléotide, comme décrit par exemple par Dreyer et al. (Proc. Natl. Acad. Sci., 82 : 968-972 ; 1985). Un groupement EDTA est couplé sur une thymidine. En présence de DTT, de Fe(II) et 02, il se produit un clivage de l'ADN au niveau de la thymidine portant l'EDTA.

Comme il est possible de coupler à l'oligonucléotide de détection une biotine, pour une fixation ultérieure sur une avidine, on peut aussi coupler un groupement chimique du type homo-bifonctionnel ou hétéro-bifonctionnel, (fonctions chimiques nécessaires à sa fixation sur l'oligonucléotide et sur le support) qui contient une fonction chimique supplémentaire qui peut être clivée sous certaines conditions : par exemple un groupement photo-clivable ou encore un groupement réduit par le DTT. Dans le cas d'un groupement photo-clivable, l'exposition de l'oligonucléotide à une longueur d'onde donnée clive la fonction et dissocie l'oligonucléotide de son support.

Une autre méthode peut également consister à ajouter une séquence connue de petite taille, dite « tag », qui a la propriété de se chélater à un groupement chélatant présent sur la nanoparticule, via un ion métallique. Le tag peut être un tag Histidine ou tout autre molécule décrite par l'état de l'art. Le groupement chélatant peut être un NTA (Nitrilotriacetic acid) ou tout autre groupement décrit par l'état de l'art. La dissociation va consister à utiliser les méthodes décrites par l'état de l'art pour dissocier l'interaction tag-métal-groupement chelatant (par exemple utilisation d'EDTA).

La dissociation peut être également effectuée par déplacement de la sonde de détection, présente sur la nanoparticule, par des oligonucléotides de séquences identiques. Ces oligonucléotides peuvent avoir soit une séquence plus courte ou la même séquence avec une séquence supplémentaire complémentaire de la cible. Egalement, il est possible d'utiliser des analogues des acides nucléiques pour ce déplacement par compétition, par exemples des PNAs (Peptide Nucleic Acids) ou autre analogue présentant l'avantage d'un squelette neutre.

En ce qui concerne la dissociation de molécules protéiques tels que des antigènes et/ou des anticorps, il est possible d'appliquer toutes les méthodes décrites dans l'état de l'art pour dissocier les interactions entre un antigène et un anticorps (nombreuses techniques connues dans le domaine de la chromatographie d'affinité). A titre d'exemple, il est ainsi possible d'effectuer la dissociation en appliquant des solutions acides telles qu'un tampon glycine à pH acide ou encore des solutions à force ionique élevée telles que le LiCl 5M (Kubitschko et al., Anal. Biochem. 253(1):112-122 ; 1997). Il est également possible d'utiliser des agents dénaturants du type du chlorure de guanidium, urée ou encore des solutions contenant des détergents ; ou des méthodes de digestion des protéines par des protéases voire des endoprotéases spécifiques de l'antigène ou de l'anticorps ou encore non spécifique des antigènes ou des anticorps.

Dans le cas d'un double marquage, la dissociation doit être orientée, sans être dénaturante pour les protéines de façon à permettre un deuxième marquage. Ceci peut par exemple être réalisé par déplacement par l'utilisation de peptides de synthèse, dont la séquence correspond à la séquence des épitopes des anticorps monoclonaux mais avec une affinité supérieure.

Il est également possible de modifier, chimiquement ou par les techniques de génie génétique, les antigènes et les anticorps pour effectuer la dissociation sous certaines conditions. Ces méthodes ne concernent toutefois pas les molécules protéiques qui interagissent directement avec la surface de façon non spécifique. Ainsi, la modification peut porter sur une des protéines (antigène ou anticorps) en insérant par exemple par génie génétique une séquence de clivage par une endoprotéase. En présence de l'enzyme, il y a clivage de la protéine à détecter. Le deuxième marquage se fait en utilisant un deuxième anticorps monoclonal spécifique de la séquence protéique restante. La modification peut porter sur l'extrémité de la protéine en ajoutant par exemple par génie génétique un tag. Ce tag peut être reconnu par des anticorps monoclonaux, la dissociation se fait alors par déplacement à l'aide de peptides ou anticorps compétiteurs. A titre indicatif, le tag ajouté peut être une séquence « poly-Histidine », peut contenir une séquence de clivage par des endoprotéases qui n'existe pas dans la séquence, la dissociation se fait alors par clivage enzymatique. D'autres séquences telles que des séquences de « splice » de protéines peuvent également être intégrées dans la protéine. Le clivage se fait en présence de DTT par exemple.

Enfin, il est aussi possible d'ajouter à la protéine de détection une séquence d'acide nucléique. On obtient alors un tag, comme ci-dessus, pouvant être reconnu par une sonde nucléique elle-même conjuguée à une nanoparticule. La dissociation, orientée ou non, peut alors se faire par une des techniques décrites pour les acides nucléiques, sous réserve de ne pas dénaturer l'ensemble protéique dans le cas d'une dissociation orientée. Les méthodes de compétition sont avantageuses de ce point de vue.

Tous ces éléments évoqués ci-dessus sont susceptibles d'être incorporés dans l'invention afin d'améliorer encore les performances de celle-ci.

Selon la demande de brevet WO-A-99/65926, de l'une des Demanderesses, il est également possible de se passer de la molécule de détection proprement dite dans le cas des acides nucléiques. Chaque cible peut être clivée chimiquement, de manière enzymatique ou physiquement, tout en subissant un marquage simultané ou non. La présence d'une molécule de détection n'est alors plus nécessaire, puisque la molécule cible est marquée.

Il existe des dispositifs de lecture des molécules marquées ou non, susceptibles d'être présentes dans les zones de reconnaissance de la puce.

La lecture des zones de reconnaissance peut en effet être réalisée également sans la présence de marqueur, une telle technologie étant déjà bien connue de l'état de la technique.

Parmi les méthodes directes de détection d'hybridation, on peut distinguer notamment la détection de la variation de la masse, de la variation d'épaisseur et de la variation d'indice. On connaît également des méthodes photothermiques qui sont décrites dans le document de S. E. Bialkowski, vol. 137, sous le titre : « Photothermal spectrocopy methods for chemical analysis » tiré de Chemical analysis : a serie of monographs on analytical chemistry and its application, Wiley. Enfin, le document US-A-4,299,494 décrit une technique de déflexion photothermique.

Ainsi l'invention trouve des applications dans les domaines de l'analyse biologique et chimique.

En général, la lecture des diagnostics moléculaires ou immunologiques ci-dessus évoqués présente l'inconvénient essentiel d'être limité par les faux positifs, c'est-à-dire des hybrides ou complexes qui se forment alors qu'ils n'auraient pas dû s'hybrider ou se complexer, et/ou les faux négatifs, c'est-à-dire des hybrides ou complexes qui ne se forment pas alors qu'ils auraient dû s'hybrider ou se complexer. Cette présence de faux positifs ou de faux négatifs entraîne un autre inconvénient, qui est la conséquence du premier inconvénient évoqué au-dessus ; les diagnostics moléculaires ou les dosages immunologiques manquent de sensibilité (pouvoir de mettre en évidence l'hybride ou le complexe recherché lorsque celui-ci est présent en faible quantité dans un échantillon biologique à tester) et/ou de spécificité (pouvoir de détecter l'hybride ou le complexe recherché dans l'échantillon biologique à tester contenant d'autres hybrides ou complexes). Ceci peut entraîner des erreurs de diagnostic qui ne sont pas admissibles pour les patients, les praticiens et les sociétés fabriquant de tels tests.

La présente invention propose de résoudre l'ensemble des inconvénients de l'état de la technique ci-dessus mentionnés en proposant un procédé de lecture qui soit peu ou pas influencé par la présence de faux positifs et qui donc améliore très sensiblement la sensibilité et la spécificité des tests diagnostics.

A cet effet, la présente invention concerne selon un premier mode de réalisation un procédé de lecture sur un support d'au moins une réaction biologique, selon la revendication 2.

Selon un deuxième mode de réalisation, la présente invention concerne un procédé de lecture sur un support d'au moins une réaction biologique, selon la revendication 3.

Dans les deux cas de figure précédents, le procédé peut comporter en outre les étapes ultérieures supplémentaires suivantes :
- mettre en contact le support fonctionnalisé et traité, après séparation spécifique, avec le deuxième échantillon liquide ou avec un autre échantillon liquide, afin de fixer à nouveau au moins une molécule biologique cible marquée sur :
   ■ la ou les molécules biologiques de reconnaissance dont elle ou elles avaient été séparées, et/ou
   ■ toute(s) autre(s) molécules de reconnaissance issue(s) de la même zone de reconnaissance,
- réaliser une troisième image dudit support après cette nouvelle fixation de molécule(s) cible(s),
- analyser la troisième image par rapport à l'analyse précédente des deux premières images pour confirmer les fixations spécifiques entre les molécules de reconnaissance et cible(s).

Selon un troisième mode de réalisation, la présente invention concerne un procédé de lecture sur un support d'au moins une réaction biologique, selon la revendication 5.

Selon un quatrième mode de réalisation, la présente invention concerne un procédé de lecture sur un support d'au moins une réaction biologique, selon la revendication 6.

Selon un cinquième mode de réalisation, la présente invention concerne un procédé de lecture sur un support d'au moins une réaction biologique, selon la revendication 7.

Selon un sixième mode de réalisation, la présente invention concerne un procédé de lecture sur un support d'au moins une réaction biologique, selon la revendication 8.

Selon un septième mode de réalisation, la présente invention concerne un procédé de lecture sur un support d'au moins une réaction biologique selon la revendication 9.

Selon un huitième mode de réalisation, la présente invention concerne un procédé de lecture sur un support d'au moins une réaction biologique selon la revendication 10.

Dans les cinq cas de figure précédents, le procédé peut comporter les étapes ultérieures supplémentaires suivantes :
- mettre en contact le support fonctionnalisé et traité, après séparation spécifique, avec le troisième échantillon liquide ou avec un autre échantillon liquide, afin de fixer à nouveau au moins une molécule de détection marquée sur :
   ■ la ou les molécules cibles dont elle ou elles avaient été séparées, et/ou
   ■ toute(s) autre(s) molécule(s) cible(s) (3) fixée(s) à une ou des molécules de reconnaissance (2) issue(s) de la même zone de reconnaissance,
- réaliser une troisième image dudit support après cette nouvelle fixation de molécule(s) de détection,
- analyser la troisième image par rapport à l'analyse précédente des deux premières images pour confirmer les fixations spécifiques entre les molécules cible(s) et de détection.

Selon une première variante de réalisation de l'invention, au moins un lavage est réalisé après fixation :
- des molécules de reconnaissance sur le support, les molécules de reconnaissance étant éventuellement fixées à des molécules cibles marquées et/ou à des molécules cibles non marquées, ces dernières étant éventuellement fixées à des molécules de détection, et/ou
- des molécules cibles marquées et/ou des molécules cibles non marquées sur les molécules de reconnaissance, elles-mêmes préalablement fixées sur ledit support, les molécules cibles étant éventuellement fixées à des molécules de détection, et/ou
- des molécules de détection sur des molécules cibles non marquées, celles-ci étant fixées sur des molécules de reconnaissance, elles-mêmes étant fixées sur le support.

Selon une deuxième variante de réalisation de ladite invention, le support est constitué par des particules magnétiques, et le procédé comporte des étapes d'aimantation desdites particules magnétiques, qui sont préférentiellement mises en place :
- durant toute(s) étape(s) de traitement physico-chimique et/ou de réalisation d'image, telle(s) que décrite(s) précédemment, et/ou
- avant toute étape de lavage, telle que décrite précédemment.

Selon une troisième variante de réalisation de l'invention, la mise en contact du support avec au moins deux premiers échantillons liquides différents, éventuellement préalablement mélangés, permet la fixation d'au moins deux molécules de reconnaissance différentes sur ledit support en au moins deux zones de reconnaissance distinctes.

Quelle que soit la variante, lorsque l'on souhaite quantifier au moins deux molécules cibles éventuellement différentes, la molécule de détection est constituée par une molécule associée, directement ou indirectement à au moins un marqueur, telle qu'une nanoparticule, dont les moyens, qui réalisent les images, permettent la visualisation individuelle, nonobstant la présence d'autres molécules de détection avoisinantes et similaires.

Dans tous les cas de figure, les conditions physico-chimiques permettant la séparation des molécules cibles marquées par rapport aux molécules de reconnaissance ou des molécules de détection par rapport aux molécules cibles peuvent être obtenues par un chauffage à un point de fusion.

Selon un mode préférentiel de réalisation, les molécules cibles marquées, les molécules de reconnaissance ou les molécules cibles sont constituées par ou les molécules de détection comportent des acides nucléiques.

Selon un autre mode préférentiel de réalisation, les molécules cibles marquées, les molécules de reconnaissance ou les molécules cibles sont constituées par ou les molécules de détection comportent des anticorps et/ou des antigènes.

Quoiqu'il en soit le marquage des molécules cibles ou des molécules de détection peut être réalisé par :
- des particules susceptibles d'être visualisées par microscopie optique conventionnelle, microscopie à fluorescence, microscopie à fond noir, microscopie à force atomique,
- des molécules susceptibles d'être visualisées par microscopie à force atomique,
- des enzymes à produit précipitant qui produisent un signal détectable par exemple par fluorescence, luminescence, comme la péroxydase de raifort, la phosphatase alcaline, la β-galactosidase, la glucose-6-phosphate déshydrogénase,
- des chromophores comme les composés fluorescents, luminescents,
- des marqueurs électroniques détectable par microscopie électronique,
- des molécules absorbantes susceptibles d'être visualisées par microscopie à lentille thermique.

Dans le cas où l'on réalise trois images successives du support, l'autre échantillon liquide, en lieu et place soit du deuxième soit du troisième échantillon liquide, pour la mise en contact du support fonctionnalisé et traité, après séparation spécifique, comporte un marqueur différent de celui contenu par ledit deuxième ou troisième échantillon liquide.

Les figures et exemples ci-joints sont donnés à titre d'exemple explicatif et n'ont aucun caractère limitatif. Ils permettront de mieux comprendre l'invention.
La figure 1 représente une étape du procédé de lecture consistant en l'introduction d'un deuxième liquide contenant des molécules cibles au sein d'une biopuce qui porte pour sa part des molécules de reconnaissance.
La figure 2 représente une étape du procédé de lecture consistant en un lavage pour éliminer les molécules cibles non hybridées ou non complexées, c'est-à-dire qui ne se sont pas fixées au sein de la biopuce.
La figure 3 représente une étape du procédé de lecture consistant en l'introduction d'un troisième liquide contenant des molécules de détection au sein d'une biopuce qui porte à la fois des molécules de reconnaissance et des molécules cibles.
La figure 4 représente une étape du procédé de lecture consistant en un lavage pour éliminer les molécules de détection non hybridées ou non complexées, c'est-à-dire qui ne se sont pas fixées au sein de la biopuce.
La figure 5 représente une étape du procédé de lecture consistant en la réalisation d'une première image qui représente les hybrides ou complexes détectés sur la biopuce après les étapes représentées aux figures 1 à 4 précédentes.
La figure 6 représente une étape du procédé de lecture consistant en un clivage orientée permettant la dissociation entre les molécules cibles et les molécules de détection.
La figure 7 représente une étape du procédé de lecture consistant en la réalisation d'une deuxième image qui représente les hybrides ou complexes détectés sur la biopuce après l'étape représentée à la figure 6 précédente.
La figure 8 représente une étape du procédé de lecture consistant en la réintroduction du troisième liquide contenant des molécules de détection au sein d'une biopuce qui porte à la fois des molécules de reconnaissance et des molécules cibles.
La figure 9 représente une étape du procédé de lecture consistant en un lavage pour éliminer les molécules de détection qui ne se sont pas fixées au sein de la biopuce.
La figure 10 représente une étape du procédé de lecture consistant en la réalisation d'une troisième image qui représente les hybrides ou complexes détectés sur la biopuce après l'étape représentée aux figures 8 et 9 précédentes.

Enfin, la figure 11 représente la dernière étape du procédé de lecture selon l'invention, cette étape consiste en l'analyse conjointe des deuxième et troisième images pour obtenir une quatrième image qui est réellement représentative des hybrides ou complexes détectés sur la biopuce.

Les exemples présents ci-dessus permettront également de mieux comprendre l'invention.

### Exemple 1 : Dissociation spécifique des molécules de détection associées à des nanoparticules magnétiques vis-à-vis de molécules cibles :

Le modèle, choisi parmi les séquences HIV est constitué de:
- une molécule de reconnaissance 2, biotinylée en 5', pour permettre sa fixation sur un support 1, correspondant à la séquence SEQ ID N°1 : 5'-TCACTATTAT CTTGTATTAC TACTGCCCCT TCACCTTTCC AGAGGAGCTT TGCTGGTCCT TTCCAAAGTG-3' (longueur : 70 nucléotides),
- une molécule cible 3, correspondant à la séquence SEQ ID N°2 : 5'-ACAGCAGTAC AAATGGCAGT ATTCATCCAC AATTTTAAAA GAAAAGGGGG GATTGGGGGG TACAGTGCAG GGGAAAGAAT AGTAGACATA ATAGCAACAG ACATACAAAC TAAAGAATTA CAAAAACAAA TTACAAAAAT TCAAAATTTT CGGGTTTATT ACAGGGACAG CAGAAATCCA CTTTGGAAAG GACCAGCAAA GCTCCTCTGG AAAGGTGAAG GGGCAGTAGT AATACAAGAT AATAGTGACA TAAAAGTAGT GCCAAGAAGA AAAGCAAAGA TCATTAGGGA TTATGGAAAA CAGATGGCAG GTGATGATTG TGTGGCAAGT AGACAGGATG AGATTAGAAC ATGGAAAAGT TTAGTAAAAC ACCATATGTA TGTTTCAGGG AAAGCTAGGG GATGGTTTTA TAGACATCAC TATGAAAGCC CTCATCCAAG AATAAGTTCA GAAGTAAATC GAATTCCCGC GGCCATGGCG GCCGGGAGCA TGCGACGTCG GGCCCAATTC GCCC-3' (longueur : 514 nucléotides), et
- une molécule de détection 4, comprenant un oligonucléotide biotinylé au niveau de son extrémité 3', correspondant à la séquence SEQ ID N°3 : 5'-TTCTGAACTT ATTCTT-3' (longueur : 16 nucléotides), et un marqueur.

Au niveau de la SEQ ID N°2, une partie de la séquence est représentée en gras et soulignée, cette partie correspond à la séquence complémentaire de la SEQ ID N°1 de la molécule de reconnaissance 2. Une autre partie de la séquence est représentée uniquement soulignée, cette partie correspond à la séquence complémentaire de la SEQ ID N°3 de la molécule de détection 4.

### Première étape : Immobilisation des molécules de reconnaissance sur le support :

Le support 1 utilisé est une lame de verre Corning (référence 0211) format 18 mm x 18 mm, préalablement silanisée par l'AMPMES 1% (Amino-propyl-diméthyléthoxysilane).

Sur ce support 1 est greffée la neutravidine (Neutravidin biotin-binding Protein Pierce référence 31000AH) via le PDC (Phénylène diisothiocyanate) à une concentration de 1 mg/ml de PBS (Phosphate Buffer Saline) sous forme de dépôt de 2 µl soit 2 mm de diamètre. Cette étape préalable, non représentée sur les figures, permet la fixation ultérieure des molécules de reconnaissance 2 sur le support 1, par immobilisation de l'extrémité biotinylée desdites molécules de reconnaissance 2 à la neutravidine présente sur ledit support 1.

Après lavage par une solution d'ammoniaque 1 %, NaCl 1M, BSA 1 % (Bovin Serum Albumin) et incubation dans ce même tampon pendant 10 minutes, le support 1 est lavé à l'eau puis en tampon TE (Tris 10 mM à pH 8, EDTA 1 mM), NaCl 1M, afin d'éliminer la neutravidine non fixée.

Le support 1 sur lequel est fixée la neutravidine est ensuite incubé pendant 20 minutes à température ambiante en présence des molécules de reconnaissance 2, en solution dans du TE, NaCl 1 M, à une concentration de 5 µM. On lave le support 1 dans une solution d'ammoniaque à 1 %, NaCl 1 M, BSA 1 % par une incubation de 10 minutes. Ce support 1 est ensuite plongé dans cette même solution afin de saturer la totalité des zones de reconnaissance, pendant 20 minutes, lavé à l'eau puis en TE, afin de favoriser l'élimination des molécules de reconnaissance 2 non fixées à la neutravidine.

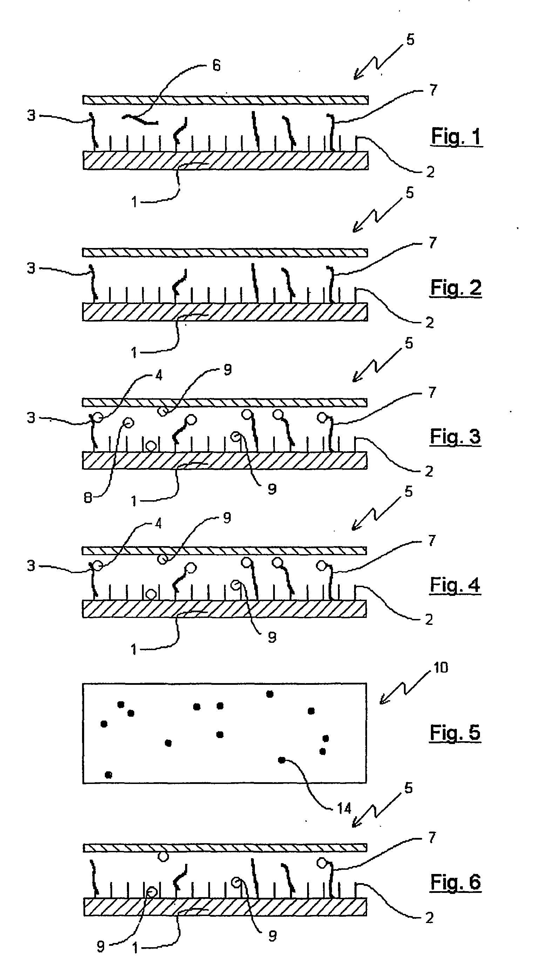

L'étape de fixation des molécules de reconnaissance 2 sur le support 1 via leur extrémité 5' biotynilée n'est pas représentée sur les figures, puisque le support 1, selon la figure 1, est déjà fonctionnalisé pour recevoir les molécules cibles 3.

### Deuxième étape : Hybridation des molécules cibles aux molécules de reconnaissance :

Après immobilisation des molécules de reconnaissance 2, l'hybridation de celles ci 2 avec les molécules cible 3 est réalisée par incubation du support en TE NaCl 1M, Triton X100 0,05% pendant une nuit à 35°C ce qui correspond à la figure 1. Dans le cas présent, 70 paires de bases complémentaires sont hybridées.

Le support 1 est ensuite repris, lavé dans ce même tampon pendant 15 minutes à température ambiante, comme représentée sur la figure 2, ce qui permet d'éliminer les molécules cibles 6 non hybridées.

Il est intéressant de noter que selon une variante de l'invention, les molécules de reconnaissance 2 peuvent être préalablement hybridées avec les molécules cibles 3 et le mélange est ensuite mis en contact directement avec le support 1.

### Troisième étape : Marquage des oligonucléotides par des nanoparticules pour synthétiser les molécules de détection :

Afin de détecter les molécules cibles 3 hybridées aux molécules de reconnaissance 2, des molécules de détection 4 sont préalablement marquées par des nanoparticules magnétiques de chez Immunicon (Huntingdon Valley, USA ; réf. : F3106). Ces nanoparticules ont un diamètre de 145 nm et sont fonctionnalisées par de la streptavidine, généralement 6000 à 20000 molécules de streptavidines sont fixées sur une unique nanoparticule, chaque streptavidine permettant la fixation de quatre biotines. Ces nanoparticules sont incubées à une concentration de 10⁹ particules/ml à 4°C pendant une nuit dans du tampon TE NaCl 1 M, ADN de Saumon 0,14 mg/ml, Triton X100 0,05%. L'oligonucléotide, à la base de la molécule de détection 4, biotinylé sur une de ses extrémités, est ensuite ajouté à la solution de nanoparticules, à raison de mille oligonucléotides de détection par nanoparticule, puis incubé 30 minutes à 35°C.. Chaque oligonucléotide de détection associé à une nanoparticule constitue une molécule de détection 4.

### Quatrième étape : 1^{ère} hybridation des molécules de détection aux molécules cibles :

Cette étape, représentée sur la figure 3, consiste à mettre en contact les molécules cibles 3 hybridées aux molécules de reconnaissance 2 avec les molécules de détection 4. Pour cela, le support 1, sur lequel sont fixées les molécules cibles 3 hybridées aux molécules de reconnaissance 2, est incubé avec 1 ml de la solution de molécules de détection 4, préparées selon l'étape précédente, à raison de 10⁹ nanoparticules / ml pendant 1 heure à température ambiante. L'hybridation s'effectue sur 16 paires de bases complémentaires.

Le support 1 est ensuite lavé dans du tampon TE, NaCl 1M, Triton X100 0,05% pendant 15 minutes à température ambiante, afin de favoriser l'élimination des molécules de détection non hybridées sur les molécules cibles 3, comme représenté sur la figure 4.

Il est intéressant de noter que selon une variante de l'invention, les molécules de détection 4 peuvent être préalablement hybridées avec les molécules cibles 3 et le mélange est ensuite mis en contact directement avec le support 1 sur lequel sont fixées les molécules de reconnaissance 2.

### Cinquième étape : Obtention d'une 1^{ère} image :

On réalise une première image 10 en microscopie en fond noir (grossissement de vingt (20) fois avec objectif à fond noir) qui permet de visualiser des nanoparticules de petite taille non fluorescentes, ce qui correspond à la figure 5.

### Sixième étape : Dissociation thermique des molécules cibles et des molécules de détection :

Cette étape de dissociation est représentée sur la figure 6. La molécule de détection 4 possède un point de fusion de 51°C alors que la molécule de reconnaissance 2 a un point de fusion mesuré de 90,5°C. Une température de 60°C constitue une température supérieure au point de fusion de la molécule de détection 4 et inférieure au point de fusion de la molécule de reconnaissance 2, permettant une dissociation entre la molécule cible 3 et la molécule de détection 4. La dissociation des molécules de détection 4 est ainsi réalisée par traitement du support 1 en tampon TE, Triton X100 0,05% à 60°C pendant 1 heure. Certaines molécules de détection 4 restent sur la surface, mais celles qui restent correspondent aux molécules de détection adsorbées non spécifiquement sur la surface

Cette dissociation peut également être réalisée par incubation du support 1 en tampon Phosphate de Sodium à pH 5,5 à une concentration de 100 mM, EDTA 1 mM, Triton X100 0,05% pendant 1 heure à 60°C. La dissociation ne dépend pas de la nature du tampon utilisé pour l'hybridation (tampon Phosphate de Sodium à pH 5,5 et 100 mM, NaCl 1 M, EDTA 1 mM, Triton X100 0,05% ou tampon Phosphate de Sodium à pH 5,5 et 100 mM, NaCl 0,1 M, EDTA 1 mM, Triton X100 0,05%). Les conditions nécessaires sont l'utilisation d'un tampon basse force ionique, voire sans sel, et le chauffage pendant 1 heure à 60°C.

### Septième étape : Obtention d'une deuxième image :

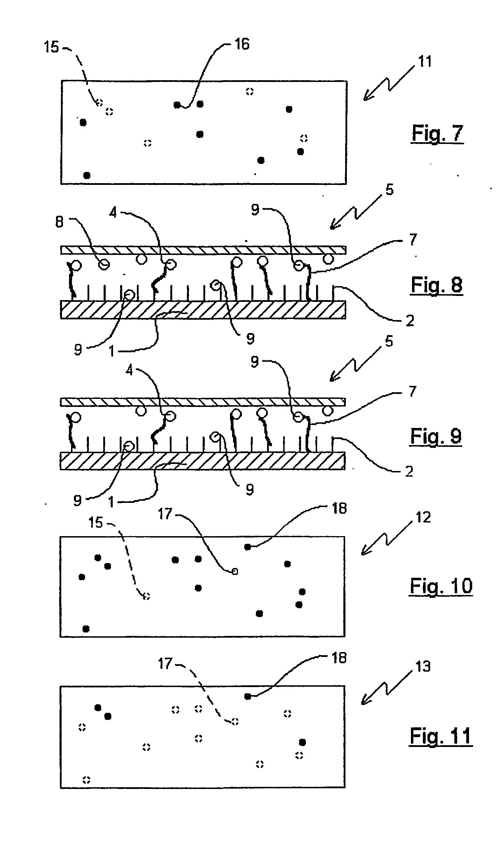

On réalise en microscopie en fond noir, une deuxième image 11 qui permet d'observer que la plupart des molécules de détection 4 préalablement hybridés aux molécules cibles, qui constituaient de vrais positifs a disparu. C'est ce qui est représenté sur la figure 7.

Cette 2^{ème} image 11 permet ainsi de détecter les molécules de détection 4 qui sont seulement adsorbées sur la surface ou qui n'étaient pas convenablement hybridées aux molécules cibles 3, c'est à dire sans mésappariement, ce qui constituent des faux positifs.

Par soustraction des spots de détection présents sur la 1^{ère} image 10 à ceux de la 2^{ème} image 11, on peut distinguer ainsi la présence de vrais positifs et de faux positifs.

### Huitième étape : 2^{ème} hybridation des molécules de détection aux molécules cibles :

Dans l'objectif d'affiner encore les résultats des étapes précédentes, le support 1 est incubé une deuxième fois avec les molécules de détection 4 dans des conditions identiques à la première hybridation préalablement décrite dans la quatrième étape, afin de reconstituer le marquage des molécules cibles 3, déjà hybridées avec les molécules de reconnaissance 2, par les molécules de détection 4. C'est ce qui est bien représenté sur la figure 8.

Le support 1 est ensuite lavé dans du tampon TE, NaCl 1 M, Triton X100 0,05% pendant 15 minutes à température ambiante, ce qui correspond à la figure 9.

### Neuvième étape : Obtention d'une 3^{ème} image :

Cette 3^{ème} image 12 permet d'affiner la distinction faite lors de la septième étape entre les vrais positifs et les faux positifs. De plus, les molécules de détection 4 étant spécifiques des molécules cibles 3, cette 3^{ème} image 12 permet de vérifier, comme représenté sur la figure 10, que lesdites molécules cibles 3 sont toujours présentes sur lesdites molécules de reconnaissance 2, puisque l'on retrouve la même intensité du signal de détection que celle de la 1^{ère} image. Par conséquent, les molécules cibles 3 ne se sont pas dissociées des molécules de reconnaissance 2 lors du traitement par le TE, Triton X100 à 60°C: la dissociation est orientée.

Il est également possible de réaliser une ultime étape correspondant à la figure 11, où des moyens informatiques peuvent traiter les trois images obtenues 10, 11 et 12, afin de définir précisément les spots de détection 18 correspondant réellement à des molécules de détection 4 hybridées sur des hybrides molécules de reconnaissance 2 - molécules cibles 3.

### Exemple 2 : Dissociation orientée des molécules de détection associées à des nanoparticules fluorescentes vis-à-vis de molécules cibles :

Cet exemple utilise le modèle biologique décrit dans l'exemple 1.

Les deux premières étapes décrites dans l'exemple 1, qui correspondent respectivement à l'immobilisation des molécules de reconnaissance 2 sur le support 1, et l'hybridation des molécules cibles 3 aux molécules de reconnaissance 2 sont réalisées d'une façon comparable dans cet exemple.

La troisième étape, qui correspond au marquage des molécules de détection diffère par contre de l'exemple 1 puisque les nanoparticules qui ont été utilisées pour marquer les molécules de détection 4 sont des nanoparticules fluorescentes fournies par Molecular Probe (Eugene OR USA réf. : T8860) de 100 nm de diamètre, déjà fonctionnalisées par de la neutravidine. Les molécules de détection 4 ainsi obtenues sont purifiées sur Microcon YM30 (Amicon MILLIPORE réf. : 42409).

La 1^{ère} hybridation des molécules de détection 4 aux molécules cibles est réalisée comme décrit dans la quatrième étape de l'exemple 1.

Le comptage des spots de détection s'effectue par l'intermédiaire d'une 1^{ère} image 10, obtenue par microscopie à fluorescence selon la figure 5.

L'étape de dissociation est réalisée en tampon TE Triton X100 0,05% à 60°C pendant une heure, comme représentée sur la figure 7, comme décrit préalablement dans la sixième étape de l'exemple 1.

Une deuxième image 11 est réalisée par microscopie à fluorescence. Par soustraction de l'intensité de la fluorescence obtenue dans la première image à celle obtenue dans la deuxième image, on peut en déduire l'intensité de fluorescence due à la présence de faux positifs. D'une façon comparable à ce qui est décrit dans l'exemple 1, une 2^{ème} hybridation des molécules de détection 4 aux molécules cibles 3 suivie d'une troisième image 12 permet de contrôler que l'on retrouve bien une intensité de fluorescence comparable à celle détectée dans la première image 10, démontrant une fois de plus que la dissociation est bien orientée.

La dissociation peut donc être effectuée avec des molécules de détection 4 marquées par des nanoparticules de nature différente.

### Exemple 3 : Dissociation orientée d'une molécule cible hybridée entre deux oligonucléotides associés à des nanoparticules :

Les séquences des molécules de reconnaissance 2, molécules cibles 3 et molécules de détection 4, utilisées dans cet exemple sont identiques à celles décrites dans l'exemple 1.

Dans cet exemple, l'invention est utilisée avec un double hybride associant la molécule cible 3 à :
- la molécule de reconnaissance 2, elle-même 2 associée à une particule magnétique, et
- la molécule de détection 4 marquée par une nanoparticule fluorescente.

L'utilisation d'une particule magnétique, qui ne fait pas ici office de marqueur, permet d'utiliser un protocole simple de purification par simple aimantation, et l'utilisation d'une particule fluorescente permet d'utiliser la fluorescence comme méthode sensible de détection et de comptage des hybrides fabriqués.

Ainsi, les molécules de reconnaissance 2 sont associées à des particules magnétiques (Immunicon Streptavidine ; Hutingdon Valley, USA, réf. F3106 ; diamètre 145 nm ; concentration 10⁹ p/ml) alors que les molécules de détection 4 sont associées à des nanoparticules fluorescentes (Molecular Probes Neutravidine ; diamètre 100 nm ; concentration 10⁹ p/ml).

Dans un premier temps, les molécules cibles 3 sont hybridées aux molécules de détection 4 fluorescentes. Les molécules cibles 3 (concentration : 10⁶ p/µl) sont incubées à température ambiante pendant 4 heures avec les molécules de détection 4 (concentration : 1nM) en tampon TE NaCl 1M Triton 0,05% (volume final : 20µl).

Dans un deuxième temps, on incube pendant 1 heure à température ambiante les molécules cibles 3 hybridées aux molécules de détection 4, avec les molécules de reconnaissance 2 associées aux particules magnétiques. Les molécules cibles 3 sont ainsi prises en sandwich entre une molécule de reconnaissance 2 magnétisable et une molécule de détection 4 fluorescente.

Dans un troisième temps, les doubles hybrides sont observés au microscope en éclairage à épifluorescence ou en éclairage à fond noir. Un dispositif à aimant permanent, placé sous la platine du microscope, permet d'appliquer un champ magnétique de 300 Gauss. Sous l'action de ce champ magnétique, les particules magnétiques s'assemblent en bâtonnets dans lesquels peuvent s'incorporer des particules fluorescentes. Toutefois, à cette étape du test, on ne peut conclure si ces conjugués incorporent une molécule cible ou non.

Dans un quatrième temps, on procède, pour lever cette incertitude à une étape de dissociation orientée. Pour cela, le tampon de suspension des particules est remplacé, sous champ de confinement magnétique, par un tampon à force ionique faible TE Triton X100 0,05%, et les particules sont chauffées à 70°C pendant 30 minutes.

Immédiatement après, on effectue un lavage magnétique. La suspension obtenue est examinée au microscope toujours sous champ magnétique de 300 Gauss. La combinaison de la force ionique et de la température a donc permis de séparer les molécules cibles 3 des molécules de détection 4. Le taux de dissociation dépend de la nature exacte des fonctionnalisations des deux types de nanoparticules et aussi de la longueur et de la séquence des molécules d'ADN, et de la composition du tampon.

Ce mode d'application de dissociation orientée entre une particule fluorescente et une particule magnétique, ou plus précisément entre une molécule cible 3 et une molécule de détection 4, peut être mis en oeuvre avec d'autres types de particules non magnétiques ou non fluorescentes, ou avec des particules dont la fonctionnalisation est différente, ou avec des particules de tailles différentes.

### Exemple 4 : Dissociation orientée des molécules cibles marquées vis-à-vis de molécules de reconnaissance

La caractéristique principale de cet exemple est d'utiliser directement des molécules cibles marquées, sans avoir recours à des molécules de détection pour détecter l'hybridation entre les molécules de reconnaissance et les molécules cibles. Le modèle est constitué de :
- une molécule de reconnaissance, dont la séquence est identique à celle décrite dans l'exemple 1
- une molécule cible, dont la séquence est identique à celle décrite dans l'exemple 1, préalablement fragmentée et marquée.

Par ce procédé sont obtenus des fragments de 50 nucléotides environ, marqués en leur extrémité 3' par un marqueur fluorescent. Un de ces fragments, dit fragment de détection peut s'hybrider par complémentarité à la molécule de reconnaissance selon un protocole similaire à ce qui est décrit dans l'exemple 1.

Ainsi, l'immobilisation des molécules de reconnaissance sur le support s'effectue comme décrit dans la première étape de l'exemple 1. Après immobilisation des molécules de reconnaissance, l'hybridation des molécules de reconnaissance avec les molécules cible marquées est réalisée par incubation du support en TE NaCl 1M.

Le support 1 est ensuite repris, lavé dans ce même tampon pendant 15 minutes à température ambiante. Cette étape de lavage est très importante puisqu'elle permet d'éliminer les fragments obtenus par ce procédé qui sont les fragments de détection qui ne sont pas hybridés, et les fragments qui ne possèdent pas la région complémentaire de la molécule de reconnaissance.

On réalise une première image en microscopie à fluorescence, comme décrit dans l'exemple 2, qui permet d'obtenir le niveau de fluorescence du à la présence de vrais positifs, mais également de faux positifs.

La dissociation des molécules cibles marquées aux molécules de reconnaissance est ensuite réalisée par une incubation du support dans un tampon TE, Triton X100 0,05% à 95°C pendant 1h.

On réalise ensuite une deuxième image qui permet de détecter les fragments de détection qui sont seulement adsorbées sur la surface ou qui n'étaient pas convenablement hybridées aux molécules de reconnaissance, ce qui constituent des faux positifs. Comme décrit dans l'exemple 1, par soustraction des spots de détection présents sur la 1^{ère} image à ceux de la 2^{ème} image, on peut distinguer la présence de vrais positifs et de faux positifs.

Toujours dans l'objectif de contrôler les résultats obtenus dans les étapes précédentes, le support sur lequel sont fixées les molécules de reconnaissance est incubé une deuxième fois avec les molécules cibles dans des conditions identiques à la première hybridation molécule de reconnaissance - molécule cible préalablement décrite, et le support est lavé dans du tampon TE, NaCl 1M, Triton X100 0,05% pendant 15 minutes à température ambiante.

Une 3^{ème} image permet de vérifier la distinction faite entre les vrais positifs et les faux positifs. De plus, cette 3^{ème} image permet de vérifier que lesdites molécules de reconnaissance sont toujours présentes sur le support puisque l'on retrouve la même intensité du signal de détection que celle de la 1^{ère} image. Par conséquent, les molécules de reconnaissance ne se sont pas dissociées du support.

### REFERENCES

1. Support de la biopuce 5
2. Molécule de reconnaissance
3. Molécule cible
4. Molécule de détection
5. Biopuce
6. Molécule cible non hybridée ou non complexée
7. Molécule cible hybridée ou complexée ailleurs que sur une molécule de reconnaissance 2
8. Molécule de détection non hybridée ou non complexée
9. Molécule de détection adsorbée ou complexée ailleurs que sur une molécule cible 3
10.Première image
11. Deuxième image
12. Troisième image
13.Quatrième image
14. Spot de détection correspondant à une molécule de détection 4, 8 ou 9
15. Position d'un spot de détection ayant disparu correspondant à une molécule de détection 4
16. Spot de détection correspondant à une molécule de détection 9
17. Spot de détection venant d'apparaître correspondant à une molécule de détection 9
18. Spot de détection correspondant à une molécule de détection 4

## Revendications

1. Procédé de lecture sur un support (1) d'au moins une réaction biologique, qui consiste à :
• obtenir, fixé sur un support (1), au moins un complexe d'au moins une molécule biologique de reconnaissance (2) fixée à au moins une molécule biologique cible (3), marquée ou non marquée et, lorsque la ou lesdites molécule(s) biologique(s) cible(s) (3) n'est (ne sont) pas marquée(s), au moins une molécule de détection (4) marquée, fixée à la ou auxdites molécule(s) biologique(s) cible(s) (3) ; ledit complexe étant fixé sur le support (1) par le biais de la ou des molécule(s) biologiquc(s) de reconnaissance (2),
• réaliser une première image (10) dudit support (1),
• traiter le support dans des conditions physico-chimiques permettant la séparation de:
- chaque molécule cible (3) marquée par rapport à une molécule de reconnaissance (2), sur laquelle elle est fixée spécifiquement ou
- chaque molécule de détection (4) marquée par rapport à une molécule cible (3) non marquée,
• réaliser une deuxième image (11) dudit support (1) après cette séparation, permettant de détecter :
- la ou les molécule(s) cible(s) marquée(s) (3) qui n'est ou ne sont pas fixée(s) spécifiquement sur la ou les molécules de reconnaissance (2) ou
- la ou les molécule(s) de détection (4) marquée(s) qui n'est ou ne sont pas fixée(s) spécifiquement sur la ou les molécule(s) cible(s) (3) non marquée(s)
• analyser les deux images pour déterminer les fixations spécifiques entre la ou les molécule(s) biologique(s) de reconnaissance (2) et la ou les molécule(s) cible(s) (3) marquée(s) ou entre la ou les molécule(s) cible(s) (3) non marquée(s) et la ou les molécule(s) de détection (4) marquée(s).

2. Procédé selon la revendication 1, qui consiste à :
- mettre en contact le support (1) avec au moins un premier échantillon liquide contenant au moins un groupe de molécules biologiques de reconnaissance (2), identiques entre elles (2), afin de fixer ces molécules de reconnaissance (2) identiques sur le support (1), préférentiellement au niveau d'une zone de reconnaissance,
- mettre en contact le support fonctionnalisé avec au moins un deuxième; échantillon liquide contenant au moins une molécule biologique cible marquée, afin de fixer cette ou ces molécules cibles sur ladite ou lesdites molécules de reconnaissance (2),
- réaliser une première image (10) dudit support (1) après cette fixation de la ou des molécules cibles marquées,
- traiter le support dans des conditions physico-chimiques permettant la séparation de chaque molécule cible par rapport à une molécule de reconnaissance (2), sur laquelle elle est fixée spécifiquement,
- réaliser une deuxième image (11) dudit support (1) après cette séparation, permettant de détecter la ou les molécules cibles marquées (7) qui n'est ou ne sont pas fixées spécifiquement sur la ou les molécules de reconnaissance (2) et
- analyser les deux images pour déterminer les fixations spécifiques entre les molécules de reconnaissance (2) et cible(s).

3. Procédé selon la revendication 1, qui consiste à :
- mettre en contact au moins un premier échantillon liquide contenant au moins un groupe de molécules biologiques de reconnaissance (2), identiques entre elles, avec au moins un deuxième échantillon liquide contenant au moins une molécule biologique cible marquée, afin de fixer cette ou ces molécules cibles sur ladite ou lesdites molécules de reconnaissance,
- mettre en contact le support (1) avec le mélange des au moins deux premier et deuxième échantillons liquides contenant au moins le groupe de molécules biologiques de reconnaissance (2) identiques, molécule(s) de reconnaissance (2) éventuellement complexée(s) avec au moins une molécule cible, afin de fixer ces molécules de reconnaissance (2) ou complexe(s) sur le support (1), préférentiellement au niveau d'une zone de reconnaissance,
- réaliser une première image (10) dudit support (1) après cette fixation de la ou des molécules cibles marquées,
- traiter le support dans des conditions physico-chimiques permettant la séparation de chaque molécule cible par rapport à une molécule de reconnaissance (2), sur laquelle elle est fixée spécifiquement,
- réaliser une deuxième image (11) dudit support (1) après cette séparation, permettant de détecter la ou les molécules cibles marquées (7) qui n'est ou ne sont pas fixées spécifiquement sur la ou les molécules de reconnaissance (2) et
- analyser les deux images pour déterminer les fixations spécifiques entre les molécules de reconnaissance (2) et cible(s).

4. Procédé, selon l'une quelconque des revendications 2 ou 3, **caractérisé en ce qu'**il comporte les étapes ultérieures supplémentaires suivantes :
- mettre en contact le support fonctionnalisé et traité, après séparation spécifique, avec le deuxième échantillon liquide ou avec un autre échantillon liquide, afin de fixer à nouveau au moins une molécule biologique cible marquée sur :
- la ou les molécules biologiques de reconnaissance (2) dont elle ou elles avaient été séparées, et/ou
- toute(s) autre(s) molécule(s) de reconnaissance (2) issue(s) de la même zone de reconnaissance,
- réaliser une troisième image (12) dudit support (1) après cette nouvelle fixation de molécule(s) cible(s),
- analyser la troisième image par rapport à l'analyse précédente des deux premières images pour confirmer les fixations spécifiques entre les molécules de reconnaissance et cible(s).

5. Procédé selon la revendication 1, qui consiste à :
- mettre en contact le support (1) avec au moins un premier échantillon liquide contenant au moins un groupe de molécules biologiques de reconnaissance (2), identiques entre elles (2), afin de fixer ces molécules de reconnaissance (2) identiques sur le support (1), préférentiellement au niveau d'une zone de reconnaissance,
- mettre en contact le support fonctionnalisé avec au moins un deuxième échantillon liquide contenant au moins une molécule biologique cible (3), afin de fixer cette ou ces molécules cibles (3) sur ladite ou lesdites molécules de reconnaissance (2),
- mettre en contact le support fonctionnalisé et traité avec au moins un troisième échantillon liquide contenant au moins une molécule de détection (4) marquée, afin de fixer cette ou ces molécules de détection (4) sur ladite ou lesdites molécules cibles (3), fixées sur la ou les molécule de reconnaissance (2),
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules cible(s) et de détection.

6. Procédé selon la revendication 1, qui consiste à :
- mettre en contact au moins un premier échantillon liquide contenant au moins un groupe de molécules biologiques de reconnaissance (2), identiques entre elles, avec au moins un deuxième échantillon liquide contenant au moins une molécule biologique cible (3), afin de fixer cette ou ces molécules cibles (3) sur ladite ou lesdites molécules de reconnaissance (2),
- mettre en contact le support (1) avec le mélange des au moins deux premier et deuxième échantillons liquides contenant au moins le groupe de molécules biologiques de reconnaissance (2) identiques, molécule(s) de reconnaissance (2) éventuellement complexée(s) avec au moins une molécule cible, afin de fixer ces molécule(s) de reconnaissance (2) et/ou complexe(s) sur le support (1), préférentiellement au niveau d'une zone du reconnaissance,
- mettre en contact le support fonctionnalisé et traité avec au moins un troisième échantillon liquide contenant au moins une molécule de détection (4) marquée, afin de fixer cette ou ces molécules de détection (4) sur ladite ou lesdites molécules cibles (3), fixées sur la ou les molécule de reconnaissance (2),
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules cible(s) et de détection.

7. Procédé selon la revendication 1, qui consiste à :
- mettre en contact au moins un premier échantillon liquide contenant au moins un groupe de molécules biologiques de reconnaissance (2), identiques entre elles, avec au moins un deuxième échantillon liquide contenant au moins une molécule biologique cible (3), afin de fixer cette ou ces molécules cibles (3) sur ladite ou lesdites molécules de reconnaissance (2),
- mettre en contact le mélange des deux premier et deuxième échantillons liquides avec au moins un troisième échantillon liquide contenant au moins une molécule de détection (4) marquée, afin de fixer cette ou ces molécules de détection (4) sur ladite ou lesdites molécules cibles (3), fixées sur la ou les molécules de reconnaissance (2),
- mettre en contact le support (1) avec le mélange des au moins trois premier, deuxième et troisième échantillons liquides contenant au moins le groupe de molécules biologiques de reconnaissance (2) identiques, molécule(s) de reconnaissance (2) éventuellement complexée(s) avec au moins une molécule cible (3), elle-même (3) éventuellement complexée(s) avec au moins une molécule de détection (4), afin de fixer ces molécules de reconnaissance (2) ou complexe(s) sur le support (1), préférentiellement au niveau d'une zone de reconnaissance,
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules cible(s) et de détection.

8. Procédé selon la revendication 1, qui consiste à :
- mettre en contact au moins un deuxième échantillon liquide contenant au moins une molécule biologiques cible (3) avec au moins un troisième échantillon liquide contenant au moins une molécule biologique de détection (4), afin de fixer cette ou ces molécules cibles (3) sur la ou les molécules de détection (4),
- mettre en contact le mélange des deux deuxième et troisième échantillons liquides avec au moins un premier échantillon liquide contenant au moins un groupe de molécules de reconnaissance (2), identiques entre elles, afin de fixer cette ou ces molécules de reconnaissance (2) sur ladite ou lesdites molécules cibles (3), éventuellement fixées sur ladite ou lesdites molécules de détection (4),
- mettre en contact le support (1) avec le mélange des au moins trois premier, deuxième et troisième échantillons liquides contenant au moins le groupe de molécules biologiques de reconnaissance (2) identiques, molécule(s) de reconnaissance (2) éventuellement complexée(s) avec au moins une molécule cible (3), elle-même (3) éventuellement complexée(s) avec au moins une molécule de détection (4), afin de fixer ces molécules de reconnaissance (2) ou complexe(s) sur le support (1), préférentiellement au niveau d'une zone de reconnaissance,
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules cible(s) et de détection.

9. Procédé selon la revendication 1, qui consiste à :
- mettre en contact le support (1) avec au moins un premier échantillon liquide contenant au moins un groupe de molécules biologiques de reconnaissance (2), identiques entre elles (2), afin de fixer ces molécules de reconnaissance (2) identiques sur le support (1), préférentiellement au niveau d'une zone de reconnaissance,
- mettre en contact au moins un deuxième échantillon liquide contenant au moins une molécule biologiques cible (3) avec au moins un troisième échantillon liquide contenant au moins une molécule biologique de détection (4), afin de fixer cette ou ces molécules cibles (3) sur la ou les molécules de détection (4),
- mettre en contact le mélange des au moins deux deuxième et troisième échantillons liquides contenant au moins une molécule cible (3) éventuellement complexée avec une molécule de détection (4) afin de fixer cette ou ces molécules cibles (3) sur ladite ou lesdites molécules de reconnaissance (2),
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules ciblc(s) et de détection.

10. Procédé selon la revendication 1, qui consiste à :
- mettre en contact au moins un deuxième échantillon liquide contenant au moins une molécule biologique cible (3) avec au moins un troisième échantillon liquide contenant au moins une molécule biologique de détection (4), afin de fixer cette ou ces molécules cibles (3) sur la ou les molécules de détection (4),
- mettre en contact au moins un premier échantillon liquide contenant au moins un groupe de molécules biologiques de reconnaissance (2), identiques entre elles, avec le mélange des au moins deux deuxième et troisième échantillons liquides contenant au moins une molécule cible (3) éventuellement complexée avec une molécule de détection (4) afin de fixer cette ou ces molécules cibles (3) sur ladite ou lesdites molécules de reconnaissance (2),
- mettre en contact le support (1) avec le mélange des au moins trois premier, deuxième et troisième échantillons liquides contenant au moins un groupe de molécules biologiques de reconnaissance (2), identiques entre elles (2), molécule(s) de reconnaissance (2) éventuellement complexée(s) avec au moins une molécule cible (3), elle-même (3) éventuellement complexée avec une molécule de détection (4), afin de fixer ces molécules de reconnaissance (2) identiques sur le support (1), préférentiellement au niveau d'une zone de reconnaissance.
- réaliser une première image (10) dudit support (1) après cette fixation de la ou des molécules de détection (4 et/ou 9) marquées,
- traiter le support dans des conditions physico-chimiques permettant la séparation de chaque molécule de détection (4) par rapport à une molécule cible (3), sur laquelle elle (4) est fixée spécifiquement,
- réaliser une deuxième image (11) dudit support (1) après cette séparation, permettant de détecter la ou les molécules de détection marquées (9) qui n'est ou ne sont pas fixées spécifiquement sur la ou les molécules cibles (3) et
- analyser les deux images pour déterminer les fixations spécifiques entre les molécules cible(s) et de détection.

11. Procédé, selon l'une quelconque des revendications 5 à 10, **caractérisé en ce qu'**il comporte les étapes ultérieures supplémentaires suivantes :
- mettre en contact le support fonctionnalisé et traité, après séparation spécifique, avec le troisième échantillon liquide ou avec un autre échantillon liquide, afin de fixer à nouveau au moins une molécule de détection (4) marquée sur :
- la ou les molécules cibles (3) dont elle ou elles avaient été séparées, et/ou
- toute(s) autre(s) molécule(s) cible(s) (3) fixée(s) à une ou des molécules de reconnaissance (2) issue(s) de la même zone de reconnaissance,
- réaliser une troisième image (12) dudit support (1) après cette nouvelle fixation de molécule(s) de détection (4 et/ou 9),
- analyser la troisième image par rapport à l'analyse précédente des deux premières images pour confirmer les fixations spécifiques entre les molécules cible(s) et de détection.

12. Procédé, selon l'une quelconque des revendications 2 à 11, **caractérisé en ce qu'**au moins un lavage est réalisé après fixation :
- des molécules de reconnaissance (2) sur le support (1), les molécules de reconnaissance (2) étant éventuellement fixées à des molécules cibles marquées et/ou à des molécules cibles (3) non marquées, ces dernières (3) étant éventuellement fixées à des molécules de détection (4), et/ou
- des molécules cibles marquées et/ou des molécules cibles (3) non marquées sur les molécules de reconnaissance (2), elles-mêmes (2) préalablement fixées sur ledit support (1), les molécules cibles (3) étant éventuellement fixées à des molécules de détection (4), et/ou
- des molécules de détection (4) sur des molécules cibles (3) non marquées, celles-ci (3) étant fixées sur des molécules de reconnaissance (2), elles-mêmes (2) étant fixées sur le support (1).

13. Procédé, selon l'une quelconque des revendications 2 à 12, **caractérisé en ce que** le support (1) est constitué par des particules magnétiques, et que le procédé comporte des étapes d'aimantation desdites particules magnétiques, préférentiellement elles sont mises en place :
- durant toute étape de traitement physico-chimiquc et/ou de réalisation d'image, selon l'une quelconque des revendications 1 à 10, et/ou
- avant toute étape de lavage selon la revendication 11.

14. Procédé, selon l'une quelconque des revendications 2 à 13, **caractérisé en ce que** la mise en contact du support (1) avec au moins deux premiers échantillons liquides différents, éventuellement préalablement mélangés, permet la fixation d'au moins deux molécules de reconnaissance (2) différentes sur ledit support (1) en au moins deux zones de reconnaissance distinctes.

15. Procédé, selon l'une quelconque des revendications 2 à 14, pour quantifier au moins deux molécules cibles (3) éventuellement différentes, **caractérisé en ce que** la molécule de détection (4) est constituée par une molécule associée, directement ou indirectement à au moins un marqueur, telle qu'une nanoparticule, dont les moyens, qui réalisent les images (10, 11 et/ou 12), permettent la visualisation individuelle, nonobstant la présence d'autres molécules de détection (4 et/ou 9) avoisinantes et similaires.

16. Procédé, selon l'une quelconque des revendications 2 à 15, **caractérisé en ce que** les conditions physico-chimiques permettant la séparation des molécules cibles marquées par rapport aux molécules de reconnaissance (2) ou des molécules de détection (4) par rapport aux molécules cibles (3) sont obtenues par un chauffage a un point de fusion.

17. Procédé, selon l'une quelconque des revendications 2 à 16, **caractérisé en ce que** les molécules cibles marquées, les molécules de reconnaissance (2) ou les molécules cibles (3) sont constituées par ou les molécules de détection (4) comportent des acides nucléiques.

18. Procédé, selon l'une quelconque des revendications 2 à 16, **caractérisé en ce que** les molécules cibles marquées, les molécules de reconnaissance (2) ou les molécules cibles (3) sont constituées par ou les molécules de détection (4) comportent des anticorps et/ou des antigènes.

19. Procédé, selon l'une quelconque des revendications 2 à 18, **caractérisé en ce que** le marquage des molécules cibles ou des molécules de détection (4) est réalisé par :
- des particules susceptibles d'être visualisées par microscopie optique conventionnelle, microscopie à fluorescence, microscopie à fond noir, microscopie à force atomique,
- des molécules susceptibles d'être visualisées par microscopie à force atomique,
- des enzymes à produit précipitant qui produisent un signal détectable par exemple par fluorescence, luminescence, comme la péroxydase de raifort, la phosphatase alcaline, la β-galactosidase, la glucose-6-phosphate déshydrogénase,
- des chromophores comme les composés fluorescents, luminescents,
- des marqueurs électroniques détectables par microscopie électronique,
- des molécules absorbantes susceptibles d'être visualisées par microscopie à lentille thermique

20. Procédé, selon la combinaison de l'une quelconque des revendications 4 ou 7, d'une part, et de la revendication 18, d'autre part, **caractérisé en ce que** l'autre échantillon liquide, en lieu et place soit du deuxième soit du troisième échantillon liquide, pour la mise en contact du support fonctionnalisé et traité, après séparation spécifique, comporte un marqueur différent de celui contenu par ledit deuxième ou troisième échantillon liquide.

## Claims

1. Method of reading at least one biological reaction on a support (1), which consists in:
• obtaining, bound to a support (1), at least one complex of at least one biological recognition molecule (2) bound to at least one labelled or unlabelled biological target molecule (3) and, if said biological target molecule(s) (3) is (are) not labelled, at least one labelled detection molecule (4) bound to said biological target molecule(s) (3), said complex being bound to the support (1) via the biological recognition molecule(s) (2),
• making a first image (10) of said support (1),
• treating the support under physicochemical conditions that allow the separation of:
- each labelled target molecule (3) from a recognition molecule (2) to which it is specifically bound, or
- each labelled detection molecule (4) from an unlabelled target molecule (3),
• making a second image (11) of said support (1) after this separation, allowing the detection of:
- the labelled target molecule(s) (3) which is (are) not specifically bound to the recognition molecule(s) (2), or
- the labelled detection molecule(s) (4) which is (are) not specifically bound to the unlabelled target molecule(s) (3), and
• analysing the two images in order to determine the specific bindings between the biological recognition molecule(s) (2) and the labelled target molecule(s) (3) or between the unlabelled target molecule(s) (3) and the labelled detection molecule(s) (4).

2. Method according to Claim 1 which consists in:
- bringing the support (1) into contact with at least one first liquid sample containing at least one group of mutually identical biological recognition molecules (2) in order to bind these identical recognition molecules (2) to the support (1), preferentially in a recognition zone,
- bringing the functionalized support into contact with at least one second liquid sample containing at least one labelled biological target molecule in order to bind this (these) target molecule(s) to said recognition molecule(s) (2),
- making a first image (10) of said support (1) after this binding of the labelled target molecule(s),
- treating the support under physicochemical conditions that allow the separation of each target molecule from a recognition molecule (2) to which it is specifically bound,
- making a second image (11) of said support (1) after this separation, allowing the detection of the labelled target molecule(s) (7) which is (are) not specifically bound to the recognition molecule(s) (2), and
- analysing the two images in order to determine the specific bindings between the recognition molecules (2) and target molecule (s) .

3. Method according to Claim 1 which consists in:
- bringing at least one first liquid sample, containing at least one group of mutually identical biological recognition molecules (2), into contact with at least one second liquid sample, containing at least one labelled biological target molecule, in order to bind this (these) target molecule(s) to said recognition molecule(s),
- bringing the support (1) into contact with the mixture of the at least two first and second liquid samples containing at least the group of identical biological recognition molecules (2), said recognition molecule(s) (2) optionally being complexed with at least one target molecule, in order to bind these recognition molecules (2) or complex(es) to the support (1), preferentially in a recognition zone,
- making a first image (10) of said support (1) after this binding of the labelled target molecule(s),
- treating the support under physicochemical conditions that allow the separation of each target molecule from a recognition molecule (2) to which it is specifically bound,
- making a second image (11) of said support (1) after this separation, allowing the detection of the labelled target molecule(s) (7) which is (are) not specifically bound to the recognition molecule(s) (2), and
- analysing the two images in order to determine the specific bindings between the recognition molecules (2) and target molecule(s).

4. Method according to Claim 2 or 3, **characterized in that** it comprises the following additional subsequent steps:
- bringing the treated, functionalized support, after specific separation, into contact with the second liquid sample or with another liquid sample in order to again bind at least one labelled biological target molecule to:
- the biological recognition molecule(s) (2) from which it (they) had been separated, and/or
- any other recognition molecule(s) (2) originating from the same recognition zone,
- making a third image (12) of said support (1) after this new binding of target molecule(s), and
- analysing the third image by comparison with the previous analysis of the first two images in order to confirm the specific bindings between the recognition molecules and target molecule(s).

5. Method according to Claim 1 which consists in:
- bringing the support (1) into contact with at least one first liquid sample, containing at least one group of mutually identical biological recognition molecules (2), in order to bind these identical recognition molecules (2) to the support (1), preferentially in a recognition zone,
- bringing the functionalized support into contact with at least one second liquid sample, containing at least one biological target molecule (3), in order to bind this (these) target molecule(s) (3) to said recognition molecule(s) (2),
- bringing the treated, functionalized support into contact with at least one third liquid sample, containing at least one labelled detection molecule (4), in order to bind this (these) detection molecule(s) (4) to said target molecule(s) (3) bound to the recognition molecule(s) (2),
- making a first image (10) of said support (1) after this binding of the labelled detection molecule(s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule(s) (3), and
- analysing the two images in order to determine the specific bindings between the target molecule(s) and detection molecules.

6. Method according to Claim 1 which consists in:
- bringing at least one first liquid sample, containing at least one group of mutually identical biological recognition molecules (2), into contact with at least one second liquid sample, containing at least one biological target molecule (3), in order to bind this (these) target molecule(s) (3) to said recognition molecule(s) (2),
- bringing the support (1) into contact with the mixture of the at least two first and second liquid samples containing at least the group of identical biological recognition molecules (2), said recognition molecule(s) (2) optionally being complexed with at least one target molecule, in order to bind this (these) recognition molecule(s) (2) and/or complex(es) to the support (1), preferentially in a recognition zone,
- bringing the treated, functionalized support into contact with at least one third liquid sample, containing at least one labelled detection molecule (4), in order to bind this (these) detection molecule(s) (4) to said target molecule(s) (3) bound to the recognition molecule(s) (2),
- making a first image (10) of said support (1) after this binding of the labelled detection molecule (s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule(s) (3), and
- analysing the two images in order to determine the specific bindings between the target molecule(s) and detection molecules.

7. Method according to Claim 1 which consists in:
- bringing at least one first liquid sample, containing at least one group of mutually identical biological recognition molecules (2), into contact with at least one second liquid sample, containing at least one biological target molecule (3), in order to bind this (these) target molecule(s) (3) to said recognition molecule(s) (2),
- bringing the mixture of the two first and second liquid samples into contact with at least one third liquid sample, containing at least one labelled detection molecule (4), in order to bind this (these) detection molecule(s) (4) to said target molecule(s) (3) bound to the recognition molecule(s) (2),
- bringing the support (1) into contact with the mixture of the at least three first, second and third liquid samples containing at least the group of identical biological recognition molecules (2), said recognition molecule(s) (2) optionally being complexed with at least one target molecule (3) which itself (themselves) (3) is (are) optionally complexed with at least one detection molecule (4), in order to bind these recognition molecules (2) or complex(es) to the support (1), preferentially in a recognition zone,
- making a first image (10) of said support (1) after this binding of the labelled detection molecule(s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule(s) (3), and
- analysing the two images in order to determine the specific bindings between the target molecule(s) and detection molecules.

8. Method according to Claim 1 which consists in:
- bringing at least one second liquid sample, containing at least one biological target molecule (3), into contact with at least one third liquid sample, containing at least one biological detection molecule (4), in order to bind this (these) target molecule(s) (3) to the detection molecule (s) (4),
- bringing the mixture of the two second and third liquid samples into contact with at least one first liquid sample, containing at least one group of mutually identical recognition molecules (2), in order to bind this (these) recognition molecule(s) (2) to said target molecule (s) (3) optionally bound to said detection molecule(s) (4),
- bringing the support (1) into contact with the mixture of the at least three first, second and third liquid samples containing at least the group of identical biological recognition molecules (2), said recognition molecule(s) (2) optionally being complexed with at least one target molecule (3) which itself (themselves) (3) is (are) optionally complexed with at least one detection molecule (4), in order to bind these recognition molecules (2) or complex(es) to the support (1), preferentially in a recognition zone,
- making a first image (10) of said support (1) after this binding of the labelled detection molecule(s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule (s) (3), and
- analysing the two images in order to determine the specific bindings between the target molecule(s) and detection molecules.

9. Method according to Claim 1 which consists in:
- bringing the support (1) into contact with at least one first liquid sample, containing at least one group of mutually identical biological recognition molecules (2), in order to bind these identical recognition molecules (2) to the support (1), preferentially in a recognition zone,
- bringing at least one second liquid sample, containing at least one biological target molecule (3), into contact with at least one third liquid sample, containing at least one biological detection molecule (4), in order to bind this (these) target molecule(s) (3) to the detection molecule(s) (4),
- bringing into contact the mixture of the at least two second and third liquid samples, containing at least one target molecule (3) optionally complexed with a detection molecule (4), in order to bind this (these) target molecule(s) (3) to said recognition molecule(s) (2),
- making a first image (10) of said support (1) after this binding of the labelled detection molecule (s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule(s) (3), and
- analysing the two images in order to determine the specific bindings between the target molecule(s) and detection molecules.

10. Method according to Claim 1 which consists in:
- bringing at least one second liquid sample, containing at least one biological target molecule (3), into contact with at least one third liquid sample, containing at least one biological detection molecule (4), in order to bind this (these) target molecule(s) (3) to the detection molecule (s) (4),
- bringing at least one first liquid sample, containing at least one group of mutually identical biological recognition molecules (2), into contact with the mixture of the at least two second and third liquid samples, containing at least one target molecule (3) optionally complexed with a detection molecule (4), in order to bind this (these) target molecule(s) (3) to said recognition molecule(s) (2),
- bringing the support (1) into contact with the mixture of the at least three first, second and third liquid samples, containing at least one group of mutually identical biological recognition molecules (2), said recognition molecule(s) (2) optionally being complexed with at least one target molecule (3) which itself (3) is optionally complexed with a detection molecule (4), in order to bind these identical recognition molecules (2) to the support (1), preferentially in a recognition zone,
- making a first image (10) of said support (1) after this binding of the labelled detection molecule(s) (4 and/or 9),
- treating the support under physicochemical conditions that allow the separation of each detection molecule (4) from a target molecule (3) to which it (4) is specifically bound,
- making a second image (11) of said support (1) after this separation, allowing the detection of the labelled detection molecule(s) (9) which is (are) not specifically bound to the target molecule(s) (3), and
- analysing the two images in order to determine the specific bindings between the target molecule(s) and detection molecules.

11. Method according to any one of Claims 5 to 10, **characterized in that** it comprises the following additional subsequent steps:
- bringing the treated, functionalized support, after specific separation, into contact with the third liquid sample or with another liquid sample in order to again bind at least one labelled detection molecule (4) to:
- the target molecule(s) (3) from which it (they) had been separated, and/or
- any other target molecule(s) (3) bound to one or more recognition molecules (2) originating from the same recognition zone,
- making a third image (12) of said support (1) after this new binding of the detection molecule(s) (4 and/or 9), and
- analysing the third image by comparison with the previous analysis of the first two images in order to confirm the specific bindings between the target molecule(s) and detection molecules.

12. Method according to any one of Claims 2 to 11, **characterized in that** at least one wash is carried out after the binding of:
- the recognition molecules (2) to the support (1), the recognition molecules (2) optionally being bound to labelled target molecules and/or unlabelled target molecules (3), the latter (3) optionally being bound to detection molecules (4), and/or
- the labelled target molecules and/or unlabelled target molecules (3) to the recognition molecules (2), the latter (2) previously being bound to said support (1), the target molecules (3) optionally being bound to detection molecules (4), and/or
- the detection molecules (4) to unlabelled target molecules (3), the latter (3) being bound to recognition molecules (2) which themselves (2) are bound to the support (1).

13. Method according to any one of Claims 2 to 12, **characterized in that** the support (1) consists of magnetic particles, and **in that** the method comprises steps for the magnetization of said magnetic particles, said particles preferably being put in place:
- during any physicochemical treatment step and/or image making step according to any one of Claims 1 to 10, and/or
- before any washing step according to Claim 11.

14. Method according to any one of Claims 2 to 13, **characterized in that** the bringing of the support (1) into contact with at least two different first liquid samples, optionally mixed beforehand, allows the binding of at least two different recognition molecules (2) to said support (1) in at least two distinct recognition zones.

15. Method according to any one of Claims 2 to 14 for quantifying at least two optionally different target molecules (3), **characterized in that** the detection molecule (4) consists of a molecule directly or indirectly associated with at least one marker, such as a nanoparticle, for which the means of making the images (10, 11 and/or 12) allow individual visualization despite the presence of other neighbouring and similar detection molecules (4 and/or 9).

16. Method according to any one of Claims 2 to 15, **characterized in that** the physicochemical conditions that allow the separation of the labelled target molecules from the recognition molecules (2) or the detection molecules (4) from the target molecules (3) are obtained by heating to a melting point.

17. Method according to any one of Claims 2 to 16, **characterized in that** the labelled target molecules, the recognition molecules (2) or the target molecules (3) consist of, or the detection molecules (4) comprise, nucleic acids.

18. Method according to any one of Claims 2 to 16, **characterized in that** the labelled target molecules, the recognition molecules (2) or the target molecules (3) consist of, or the detection molecules (4) comprise, antibodies and/or antigens.

19. Method according to any one of Claims 2 to 18, **characterized in that** the labelling of the target molecules or the detection molecules (4) is effected by:
- particles visualizable by conventional optical microscopy, fluorescence microscopy, dark-field microscopy or atomic force microscopy,
- molecules visualizable by atomic force microscopy,
- precipitating-product enzymes which produce a signal detectable e.g. by fluorescence or luminescence, such as horseradish peroxidase, alkaline phosphatase, β-galactosidase or glucose-6-phosphate dehydrogenase,
- chromophores such as fluorescent or luminescent compounds,
- electronic markers detectable by electron microscopy, or
- absorbent molecules visualizable by thermal lens microscopy.

20. Method according to a combination of Claim 4 or 7, on the one hand, and Claim 18, on the other, **characterized in that** the other liquid sample, taking the place of either the second or the third liquid sample, to be brought into contact with the treated, functionalized support, after specific separation, comprises a different marker from that present in said second or third liquid sample.

## Patentansprüche

1. Verfahren zum Ablesen zumindest einer biologischen Reaktion auf einem Träger, wobei das Verfahren aus folgenden Schritten besteht:
• Erhalten, gebunden auf einem Träger (1), von zumindest einem Komplex aus zumindest einem biologischen Erkennungsmolekül (2), das an zumindest ein biologisches Zielmolekül (3) gebunden ist, und das markiert oder nicht markiert ist, und wenn das oder die biologische(n) Zielmolekül(e) (3) nicht markiert ist (sind), zumindest einem markierten Detektionsmolekül (4), das an das oder die biologische(n) Zielmolekül(e) (3) gebunden ist; wobei der Komplex auf dem Träger (1) über das oder die biologische(n) Erkennungsmolekül(e) (2) gebunden ist,
• Herstellen eines ersten Bildes (10) des Trägers (1),
• Behandeln des Trägers unter physikalisch-chemischen Bedingungen, mit welchen eine Trennung ermöglicht wird von:
- jedem markierten Zielmolekül (3) von einem Erkennungsmolekül (2), an welches es spezifisch fixiert ist, oder
- jedem markierten Detektionsmolekül (4) von einem nicht markierten Zielmolekül (3),
• Herstellen eines zweiten Bildes (11) des Trägers (1) nach dieser Trennung, das die Detektion ermöglicht von:
- dem oder den markierte(n) Zielmolekül(en) (3), das oder die nicht spezifisch an dem oder den Erkennungsmolekülen (2) gebunden ist (sind), oder
- dem oder den markierte(n) Detektionsmolekül(en) (4), die nicht spezifisch an dem oder den unmarkierte(n) Zielmoleküle(n) (3) gebunden ist (sind),
• Analysieren der zwei Bilder zur Bestimmung der spezifischen Bindungen zwischen dem oder den biologischen Erkennungsmolekül(en) (2) und dem oder den markierten Zielmolekül(en) (3) oder zwischen dem oder den unmarkierten Zielmolekül(en) (3) und dem oder den markierten Detektionsmolekül(en) (4).

2. Verfahren nach Anspruch 1, das aus den folgenden Schritten besteht:
- In-Verbindung-Bringen des Trägers (1) mit zumindest einer ersten flüssigen Probe, die zumindest eine Gruppe an biologischen Erkennungsmolekülen (2) enthält, die identisch miteinander sind, um die identischen Erkennungsmoleküle (2) auf dem Träger (1) zu binden, vorzugsweise auf der Höhe einer Erkennungszone,
- In-Kontakt-Bringen des funktionalisierten Trägers mit zumindest einer zweiten flüssigen Probe, die zumindest ein biologisches, markiertes Zielmolekül enthält, um dieses oder diese Zielmoleküle an das oder die Erkennungsmoleküle (2) zu binden,
- Herstellen eines ersten Bildes (10) des Trägers (1) nach dieser Bindung des oder der markierten Zielmoleküle,
- Behandeln des Trägers unter physikalisch-chemischen Bedingungen, die eine Trennung jedes Zielmoleküls von einem Erkennungsmolekül (2) ermöglichen, an welches dieses spezifisch gebunden ist,
- Herstellen eines zweiten Bildes (11) des Trägers (1) nach dieser Trennung, um die Detektion des oder der markierten Zielmoleküle (7) zu ermöglichen, das oder die nicht spezifisch an das oder die Erkennungsmoleküle (2) gebunden sind, und
- Analysieren der zwei Bilder zur Bestimmung der spezifischen Bindungen zwischen den Erkennungsmolekülen (2) und dem (den) Zielmolekül(en).

3. Verfahren nach Anspruch 1, das aus den folgenden Schritten besteht:
- In-Verbindung-Bringen von zumindest einer flüssigen Probe, die zumindest eine Gruppe an biologischen Erkennungsmolekülen (2) enthält, die identisch miteinander sind, mit zumindest einer zweiten flüssigen Probe, die zumindest ein biologisches markiertes Zielmolekül enthält, um dieses oder diese Zielmolekül(e) an das oder die Erkennungsmoleküle zu binden,
- In-Verbindung-Bringen des Trägers (1) mit einer Mischung aus zumindest zwei ersten und zweiten flüssigen Proben, die zumindest die Gruppe der identischen biologischen Erkennungsmoleküle (2) enthalten, sowie Erkennungsmoleküle (2), die ggf. mit zumindest einem Zielmolekül komplexiert sind, um diese Erkennungsmoleküle oder den/die Komplexe auf dem Träger zu binden, vorzugsweise auf der Höhe einer Erkennungszone,
- Herstellen eines ersten Bildes (10) des Trägers (1) nach dem Binden des oder der markierten Zielmoleküle, .
- Behandeln des Trägers unter physikalisch-chemischen Bedingungen, unter welchen die Trennung jedes Trägermoleküls von einem Erkennungsmolekül (2) ermöglicht wird, an welchem diese spezifisch gebunden sind,
- Anfertigen eines zweiten Bildes (11) des Trägers (1) nach dieser Trennung, um die Detektion des oder der markierten Zielmoleküle (7) zu ermöglichen, die nicht spezifisch an das oder die Erkennungsmoleküle (2) gebunden sind, und
- Analysieren der zwei Bilder zur Bestimmung der spezifischen Bindungen zwischen den Erkennungsmolekülen (2) und dem/den Zielmolekül(en).

4. Verfahren nach einem der Ansprüche 2 oder 3, **dadurch gekennzeichnet, dass** es die folgenden weiteren zusätzlichen Schritte aufweist:
- In-Verbindung-Bringen des funktionalisierten und behandelten Trägers, nach der spezifischen Trennung, mit einer zweiten flüssigen Probe oder mit einer anderen flüssigen Probe, um zumindest ein biologisches markiertes Zielmolekül von neuem zu fixieren an:
- das oder die biologischen Erkennungsmolekül(e) (2), von dem oder von denen sie getrennt wurden, und/oder
- irgendein anderes Erkennungsmolekül oder andere Erkennungsmoleküle (2), die von der gleichen Erkennungszone abgeleitet sind,
- Herstellen eines dritten Bildes (12) des Trägers (1) nach dieser neuen Bindung der Zielmoleküle oder des Zielmoleküls,
- Analysieren des dritten Bildes im Vergleich zur vorherigen Analyse der zwei ersten Bilder zur Bestätigung der spezifischen Bindungen zwischen den Erkennungsmolekülen und dem oder den Zielmolekül(en).

5. Verfahren nach Anspruch 1, das aus Folgendem besteht:
- In-Verbindung-Bringen des Trägers (1) mit zumindest einer ersten flüssigen Probe, die zumindest eine Gruppe an biologischen Erkennungsmolekülen (2) enthält, die identisch miteinander sind (2), um diese identischen Erkennungsmoleküle (2) auf dem Träger (1) zu binden, vorzugsweise auf der Höhe einer Erkennungszone,
- In-Verbindung-Bringen des funktionalisierten Trägers mit zumindest einer zweiten flüssigen Probe, die zumindest ein biologisches Zielmolekül (3) enthält, um dieses oder diese Zielmoleküle (3) an das oder die Erkennungsmoleküle (2) zu binden,
- In-Verbindung-Bringen des funktionalisierten und behandelten Trägers mit zumindest einer dritten flüssigen Probe, die zumindest ein markiertes Detektionsmolekül (4) enthält, um dieses oder diese Detektionsmoleküle (4) an das oder die Zielmoleküle (3) zu binden, die an das oder die Erkennungsmoleküle (2) gebunden sind,
- Herstellen eines ersten Bildes (10) des Trägers (1) nach dieser Fixierung des oder der markierten Detektionsmoleküle (4) und/oder (9),
- Behandeln des Trägers unter physikalisch-chemischen Bedingungen, unter welchen die Trennung jedes Detektionsmoleküls (4) von einem Zielmolekül (3) ermöglicht wird, an welches dieses (4) spezifisch gebunden ist,
- Anfertigen eines zweiten Bildes (11) des Trägers (1) nach dieser Trennung, um die Detektion des oder der markierten Detektionsmoleküle (9) zu ermöglichen, die nicht spezifisch an das oder die Zielmoleküle (3) gebunden sind und
- Analysieren der zwei Bilder zur Bestimmung der spezifischen Bindungen zwischen den Zielmolekülen und den Detektionsmolekülen.

6. Verfahren nach Anspruch 1, das aus den folgenden Schritten besteht:
- In-Verbindung-Bringen von zumindest einer ersten flüssigen Probe, die zumindest eine Gruppe an biologischen Erkennungsmolekülen (2) enthält, die untereinander identisch sind, mit zumindest einer zweiten flüssigen Probe, die zumindest ein biologisches Zielmolekül (3) enthält, um dieses oder diese Zielmoleküle (3) an das oder die Erkennungsmoleküle (2) zu binden,
- In-Verbindung-Bringen des Trägers (1) mit einer Mischung der zumindest zwei ersten und zweiten flüssigen Proben, die zumindest die Gruppe der identischen biologischen Erkennungsmoleküle (2) enthalten, sowie die Erkennungsmoleküle (2), die ggf. mit zumindest einem Zielmolekül komplexiert sind, um diese Erkennungsmoleküle (2) und/oder die Komplexe an den Träger (1) zu binden, vorzugsweise auf der Höhe einer Erkennungszone,
- In-Kontakt-Bringen des funktionalisierten und behandelten Trägers mit zumindest einer dritten flüssigen Probe, die zumindest ein markiertes Detektionsmolekül (4) enthält, um dieses oder diese Detektionsmoleküle (4) an das oder die Zielmoleküle (3) zu binden, die an das oder die Erkennungsmoleküle gebunden sind (2),
- Herstellen eines ersten Bildes (10) des Trägers (1) nach dieser Bindung des oder der markierten Detektionsmoleküle (4 und/oder 9),
- Behandeln des Trägers unter physikalisch-chemischen Bedingungen; unter welchen die Trennung jedes Detektionsmoleküls (4) von einem Zielmolekül (3) ermöglicht wird, an welches dieses (4) spezifisch gebunden ist,
- Herstellen eines zweiten Bildes (11) des Trägers (1) nach dieser Trennung, um die Detektion des oder der markierten Detektionsmoleküle (9) zu ermöglichen, die nicht spezifisch an das oder die Zielmoleküle (3) gebunden sind, und
- Analysieren der zwei Bilder zur Bestimmung der spezifischen Bindungen zwischen den Zielmolekülen und den Detektionsmolekülen.

7. Verfahren nach Anspruch 1, das aus Folgendem besteht:
- In-Verbindung-Bringen von zumindest einer ersten flüssigen Probe, die zumindest eine Gruppe an biologischen Erkennungsmolekülen (2) enthält, die identisch miteinander sind, mit zumindest einer zweiten flüssigen Probe, die zumindest ein biologisches Zielmolekül (3) enthält, um dieses oder diese Zielmoleküle (3) an das oder die Erkennungsmoleküle (2) zu binden,
- In-Verbindung-Bringen der Mischung der zwei ersten und zweiten flüssigen Proben mit zumindest einer dritten flüssigen Probe, die zumindest ein markiertes Detektionsmolekül (4) enthält, um dieses oder diese Detektionsmoleküle (4) an das oder die Zielmoleküle (3) zu binden, welche an das oder die Erkennungsmoleküle (2) gebunden sind,
- In-Verbindung-Bringen des Trägers (1) mit einer Mischung der zumindest drei ersten, zweiten und drei flüssigen Proben, die zumindest die Gruppe der identischen biologischen Erkennungsmoleküle (2) enthalten, sowie die Erkennungsmoleküle (2), die ggf. mit zumindest einem Zielmolekül (3) komplexiert sind, welches (3) ggf. mit zumindest einem Detektionsmolekül (4) komplexiert ist, um diese Erkennungsmoleküle (2) oder Komplexe an den Träger (1) zu binden, vorzugsweise auf der Höhe einer Erkennungszone,
- Herstellen eines ersten Bildes (10) des Trägers (1) nach dieser Bindung des oder der markierten Detektionsmoleküle (4 und/oder 9),
- Behandeln des Trägers unter physikalisch-chemischen Bedingungen, unter welchen die Trennung jedes Detektionsmoleküls (4) von einem Zielmolekül (3) ermöglicht wird, an welches dieses (4) spezifisch gebunden ist,
- Herstellen eines zweiten Bildes (11) des Trägers (1) nach dieser Trennung, um die Detektion des oder der markierten Detektionsmoleküle (9) zu ermöglichen, das oder die nicht spezifisch an das oder die Zielmoleküle (3) gebunden sind, und
- Analysieren der zwei Bilder zur Bestimmung der spezifischen Bindungen zwischen den Zielmolekülen und den Detektionsmolekülen.

8. Verfahren nach Anspruch 1, das aus folgenden Schritten besteht:
- In-Verbindung-Bringen von zumindest einer zweiten flüssigen Probe, die zumindest ein biologisches Zielmolekül (3) enthält, mit zumindest einer dritten flüssigen Probe, die zumindest ein biologisches Detektionsmolekül (4) enthält, um dieses oder diese Zielmoleküle (3) an das oder die Detektionsmoleküle (4) zu binden,
- In-Verbindung-Bringen der Mischung der zweiten und dritten flüssigen Proben mit zumindest einer ersten flüssigen Probe, die zumindest eine Gruppe an Erkennungsmolekülen (2) enthält, die identisch miteinander sind, um dieses oder diese Erkennungsmoleküle (2) an das oder die Zielmoleküle (3) zu binden, die ggf. an das oder die Detektionsmoleküle (4) gebunden sind,
- In-Verbindung-Bringen des Trägers (1) mit einer Mischung der zumindest drei ersten, zweiten und dritten flüssigen Proben, die zumindest die Gruppe der identischen biologischen Erkennungsmoleküle (2) enthalten, sowie die Erkennungsmoleküle (2), die ggf. mit zumindest einem Zielmolekül (3) komplexiert sind, welche (3) ggf. mit zumindest einem Detektionsmolekül (4) komplexiert sind, um diese Erkennungsmoleküle (2) oder Komplexe an den Träger (1) zu binden, vorzugsweise auf der Höhe einer Erkennungszone,
- Anfertigen eines ersten Bildes (10) des Trägers (1) nach dieser Bindung des oder der markierten Detektionsmoleküle (4 und/oder 9),
- Behandeln des Trägers unter physikalisch-chemischen Bedingungen, unter welchen die Trennung jedes Detektionsmoleküls (4) von einem Zielmolekül (3) ermöglicht wird, an welche diese (4) spezifisch gebunden sind,
- Anfertigen eines zweiten Bildes (11) des Trägers (1) nach dieser Trennung, um die Detektion des oder der markierten Detektionsmoleküle (9) zu ermöglichen, das oder die nicht spezifisch an die Zielmoleküle (3) gebunden sind, und
- Analysieren der zwei Bilder zur Bestimmung der spezifischen Bindungen zwischen den Zielmolekülen und den Detektionsmolekülen.

9. Verfahren nach Anspruch 1, das aus folgenden Schritten besteht:
- In-Verbindung-Bringen des Trägers (1) mit zumindest einer ersten flüssigen Probe, die zumindest eine Gruppe an biologischen Erkennungsmolekülen (2) enthält, die identisch miteinander sind, um diese identischen Erkennungsmoleküle (2) an den Träger (1) zu binden, vorzugsweise auf der Höhe einer Erkennungszone,
- In-Verbindung-Bringen von zumindest einer zweiten flüssigen Probe, die zumindest ein biologisches Zielmolekül (3) enthält mit zumindest einer dritten flüssigen Probe, die zumindest ein biologisches Detektionsmolekül (4) enthält, um dieses oder diese Zielmoleküle (3) an das oder die Detektionsmoleküle (4) zu binden,
- In-Verbindung-Bringen der Mischung aus den zumindest zwei zweiten und dritten flüssigen Proben, die zumindest ein Zielmolekül (3) enthalten, das ggf. mit einem Detektionsmolekül (4) komplexiert ist, um dieses oder diese Zielmoleküle (3) an das oder die Erkennungsmoleküle (2) zu binden,
- Anfertigen eines ersten Bildes (10) des Trägers (1) nach dieser Bindung des oder der markierten Zielmoleküle (4 und/oder 9),
- Behandeln des Trägers unter physikalisch-chemischen Bedingungen, die die Trennung jedes Detektionsmoleküls (4) von einem Zielmolekül (3) ermöglichen, an welches dieses (4) spezifisch gebunden ist,
- Anfertigen eines zweiten Bildes (11) des Trägers (1) nach dieser Trennung, um die Detektion des oder der markierten Detektionsmoleküle (9) zu ermöglichen, das oder die nicht spezifisch an das oder die Zielmoleküle (3) gebunden sind, und
- Analysieren der zwei Bilder zur Bestimmung der spezifischen Bindungen zwischen den Zielmolekülen und den Detektionsmolekülen.

10. Verfahren nach Anspruch 1, das aus folgenden Schritten besteht:
- In-Verbindung-Bringen von zumindest einer zweiten flüssigen Probe, die zumindest ein biologisches Zielmolekül (3) enthält, mit zumindest einer dritten flüssigen Probe, die zumindest ein biologisches Detektionsmolekül (4) enthält, um dieses oder diese Zielmoleküle (3) an das oder die Detektionsmoleküle (4) zu binden,
- In-Verbindung-Bringen von zumindest einer ersten flüssigen Probe, die zumindest eine Gruppe an biologischen Erkennungsmolekülen (2) enthält, die identisch untereinander sind, mit einer Mischung der zumindest zwei zweiten und dritten flüssigen Proben, die zumindest ein Zielmolekül (3) enthalten, das ggf. mit einem Detektionsmolekül (4) komplexiert ist, um dieses oder diese Zielmoleküle (3) an das oder die Erkennungsmoleküle (2) zu binden,
- In-Verbindung-Bringen des Trägers (1) mit der Mischung der zumindest drei ersten, zweiten und dritten Proben, die zumindest eine Gruppe an biologischen Erkennungsmolekülen (2) enthalten, die identisch miteinander sind, wobei die Erkennungsmoleküle (2) ggf. mit zumindest einem Zielmolekül (3) komplexiert sind, welches ggf. mit einem Detektionsmolekül (4) komplexiert ist, um die identischen Erkennungsmoleküle (2) an den Träger (1) zu binden, vorzugsweise auf der Höhe einer Erkennungszone,
- Herstellen eines ersten Bildes (10) des Trägers (1) nach dieser Bindung des oder der markierten Detektionsmoleküle (4 und/oder 9),
- Behandeln des Trägers unter physikalisch-chemischen Bedingungen, die die Trennung jedes Detektionsmoleküls (4) von einem Zielmolekül (3) ermöglichen, an welches dieses (4) spezifisch gebunden ist,
- Anfertigen eines zweiten Bildes (11) des Trägers (1) nach dieser Trennung, um die Detektion des oder der markierten Detektionsmoleküle (9) zu ermöglichen, das oder die nicht spezifisch an das oder die Zielmoleküle (3) gebunden sind, und
- Analysieren der zwei Bilder zur Bestimmung der spezifischen Bindungen zwischen den Ziel- und den Detektionsmolekülen.

11. Verfahren nach einem der Ansprüche 5 bis 10, **dadurch gekennzeichnet, dass** es die folgenden weiteren zusätzlichen Schritte aufweist:
- In-Verbindung-Bringen des funktionalisierten und behandelten Trägers, nach der spezifischen Trennung, mit einer dritten flüssigen Probe oder mit einer weiteren flüssigen Probe, um zumindest ein markiertes Detektionsmolekül (4) von neuem zu binden an:
- das oder die Zielmoleküle (3), von welchen dieses oder diese getrennt waren, und/oder
- jedes andere Zielmolekül oder Zielmoleküle (3), die an ein oder mehrere Erkennungsmoleküle (2) gebunden sind, welche von einer Erkennungszone abgeleitet sind,
- Anfertigen eines dritten Bildes (12) des Trägers (1) nach dieser neuen Bindung der Detektionsmoleküle (4 und/oder 9), und
- Analysieren des dritten Bildes im Vergleich zu der vorherigen Analyse der zwei ersten Bilder zur Bestätigung der spezifischen Bindungen zwischen den Ziel- und den Detektionsmolekülen.

12. Verfahren nach einem der Ansprüche 2 bis 11, **dadurch gekennzeichnet, dass** zumindest ein Waschschritt durchgeführt wird nach der Bindung von:
- den Erkennungsmolekülen (2) an den Träger (1), wobei die Erkennungsmoleküle (2) ggf. an die markierten Zielmoleküle gebunden sind und/oder an die unmarkierten Zielmolekülen (3), wobei die Letzteren ggf. an Detektionsmoleküle (4) gebunden sind, und/oder
- den markierten Zielmolekülen und/oder den unmarkierten Zielmolekülen, (3) an die Erkennungsmoleküle (2), welche (2) vorzugsweise an den Träger (1) gebunden sind, wobei die Zielmoleküle ggf. an die Detektionsmoleküle (4) gebunden sind, und/oder den Detektionsmolekülen (4) an die unmarkierten Zielmoleküle (3), welche (3) an die an den Träger (1) gebundenen Erkennungsmoleküle (2) gebunden sind.

13. Verfahren nach einem der Ansprüche 2 bis 12, **dadurch gekennzeichnet, dass** der Träger (1) aus magnetischen Partikeln besteht, und dass das Verfahren die Schritte der Magnetisierung der magnetischen Partikel umfasst, welche vorzugsweise platziert werden:
- während irgendeines Schrittes der physikalisch-chemischen Behandlung und/oder der Herstellung des Bildes gemäß einem der Ansprüche 1 bis 10, und/oder
- vor irgendeinem Waschschritt gemäß Anspruch 11.

14. Verfahren nach einem der Ansprüche 2 bis 13, **dadurch gekennzeichnet, dass** das In-Verbindung-Bringen des Trägers (1) mit zumindest zwei ersten flüssigen unterschiedlichen Proben, die ggf. zuvor vermengt wurden, die Bindung von zumindest zwei unterschiedlichen Erkennungsmolekülen (2) an den Träger (1) in zumindest zwei unterschiedlichen Erkennungszonen ermöglicht.

15. Verfahren nach einem der Ansprüche 2 bis 14 zur Quantifizierung von zumindest zwei Zielmolekülen (3), die sich ggf. unterscheiden, **dadurch gekennzeichnet, dass** das Detektionsmolekül (4) aus einem Molekül besteht, das entweder direkt oder indirekt mit zumindest einem Marker assoziiert ist, wie beispielsweise einem Nanopartikel, dessen Mittel, mit welchen die Bilder (10, 11 und/oder 12) hergestellt werden, die individuelle Visualisierung ermöglichen, und zwar trotz des Vorliegens benachbarter anderer Detektionsmoleküle (4 und/oder 9) und Ähnlichem.

16. Verfahren nach einem der Ansprüche 2 bis 15, **dadurch gekennzeichnet, dass** die physikalisch-chemischen Bedingungen, die die Trennung der markierten Zielmoleküle von den Erkennungsmolekülen (2), oder der Detektionsmoleküle (4) von den Zielmolekülen (3) ermöglichen, durch ein Erhitzen auf einen Schmelzpunkt gewonnen werden.

17. Verfahren nach einem der Ansprüche 2 bis 16, **dadurch gekennzeichnet, dass** die markierten Zielmoleküle, die Erkennungsmoleküle (2) oder die Zielmoleküle (3) aus Nucleinsäuren bestehen oder dass die Detektionsmoleküle (4) Nucleinsäuren aufweisen.

18. Verfahren nach einem der Ansprüche 2 bis 16, **dadurch gekennzeichnet, dass** die markierten Zielmoleküle, die Erkennungsmoleküle (2) oder die Zielmoleküle (3) aus Antikörpern und/oder Antigenen bestehen, oder dass die Detektionsmoleküle (4) Antikörper und/oder Antigene aufweisen.

19. Verfahren nach einem der Ansprüche 2 bis 18, **dadurch gekennzeichnet, dass** die Markierung der Zielmoleküle oder der Detektionsmoleküle (4) erreicht wird durch:
- Partikel, die durch eine herkömmliche optische Mikroskopie, eine Fluoreszenzmikroskopie, eine Dunkelfeldmikroskopie oder Atomic-force-Mikroskopie visualisiert werden können,
- Moleküle, die durch eine Atomic-force-Mikroskopie visualisiert werden können,
- Enzyme mit einem zu fällenden Produkt, welche ein detektierbares Signal produzieren, wie beispielsweise Fluoreszenz, Lumineszenz, wie beispielsweise Meerrettichperoxydase, alkalische Phosphatase, β-Galactosidase, Glucose-6-Phosphatdehydrogenase,
- Chromophoren wie beispielsweise fluoreszierende oder lumineszierende Verbindungen,
- elektronische Marker, die durch Elektronenmikroskopie detektierbar sind,
- absorbierende Moleküle, die durch eine Mikroskopie mit thermischen Linsen visualisiert werden können.

20. Verfahren nach einer Kombination gemäß den Ansprüchen 4 bis 7 einerseits und nach Anspruch 18 andererseits, **dadurch gekennzeichnet, dass** die andere flüssige Probe, anstelle entweder der zweiten oder der dritten flüssigen Probe einen Marker aufweist, der unterschiedlich ist zu demjenigen, der in der zweiten oder dritten flüssigen Probe enthalten ist, zum In-Verbindung-Bringen des funktionalisierten und behandelten Trägers nach der spezifischen Trennung.
